(19)

Europäisches
Patentamt
European
Patent Office
Office européen
des brevets

(11) **EP 3 923 888 B1**

(12) **EUROPEAN PATENT SPECIFICATION**

(45) Date of publication and mention
of the grant of the patent:
**13.12.2023 Bulletin 2023/50**

(21) Application number: **20756418.8**

(22) Date of filing: **21.01.2020**

(51) International Patent Classification (IPC):
**A61F 13/496** *(2006.01)*

(52) Cooperative Patent Classification (CPC):
**A61F 13/536; A61F 13/496; A61F 13/51496;**
A61F 2013/49036

(86) International application number:
**PCT/CN2020/073410**

(87) International publication number:
**WO 2020/164376 (20.08.2020 Gazette 2020/34)**

(54) **PANT-TYPE WEARABLE ARTICLE**

HOSENARTIGES KLEIDUNGSSTÜCK

ARTICLE VESTIMENTAIRE DE TYPE CULOTTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.02.2019 PCT/CN2019/075102**

(43) Date of publication of application:
**22.12.2021 Bulletin 2021/51**

(73) Proprietor: **The Procter & Gamble Company
Cincinnati, OH 45202 (US)**

(72) Inventors:
 • **MORIMOTO, Koichi
 Beijing 101312 (CN)**
 • **PEI, Ruizhi
 Beijing 101312 (CN)**
 • **ISHIHARA, Kaoru
 Cincinnati, Ohio 45202 (US)**
 • **LI, Shiqiao
 Akashi-shi, Hyogo 674--0093 (JP)**
 • **WANG, Xuechun
 Beijing 101312 (CN)**

 • **STRASEMEIER, John Andrew
 Cincinnati, Ohio 45202 (US)**
 • **IBRAHIM, Farihah
 Cincinnati, Ohio 45202 (US)**
 • **LO, Nicholas Matthew
 Cincinnati, Ohio 45202 (US)**
 • **TOURNOUX, Monica Renee
 Columbus, Ohio 43231 (US)**

(74) Representative: **P&G Patent Germany
Procter & Gamble Service GmbH
Sulzbacher Straße 40
65824 Schwalbach am Taunus (DE)**

(56) References cited:
 WO-A1-2016/029658   WO-A1-2016/048337
 WO-A1-2016/101197   WO-A1-2017/212856
 WO-A1-2017/212857   CN-A- 1 516 570
 CN-A- 102 871 802   CN-A- 109 069 323
 CN-U- 204 428 283   CN-U- 207 785 374
 JP-A- 2004 254 862   JP-A- 2015 128 573
 JP-U- 3 217 273   US-A1- 2015 282 999
 US-A1- 2017 231 837   US-A1- 2018 289 563

EP 3 923 888 B1

**Description**

FIELD OF THE INVENTION

The present invention relates to pant-type wearable articles having a favorable tactile and aesthetic sense.

BACKGROUND OF THE INVENTION

Infants and other incontinent individuals wear absorbent articles such as diapers to receive and contain urine and other body exudates. Pull-on absorbent articles, or pant-type absorbent articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult, and also for younger babies requiring a soft fit around the waist opening and leg openings.

Pant-type articles may take various structures wherein the circumference of the waist opening and vicinity thereof is made elastic enough to facilitate the wearer or the caregiver to expand the article and insert the wearer's legs into the leg openings for wearing the article. The region of the waist circumference and vicinity thereof is often referred to as the elastic belt. One type of structure for the pant-type article is the belt-type pant having a central chassis to cover the crotch region of the wearer and a separate elastic belt defining the waist opening and leg opening, such as described in PCT Publication WO 2006/17718A. Another type of structure for the pant-type article is the uni-body type pant configured such that the outer cover of the article completely covers the entirety of the garment-facing surface of the article, wherein the portion configured to stretch about the torso is considered the elastic belt region.

Whatever the structure of the article may be, the outer surface, or garment-facing surface of the article may be the portion which is most touched and observed by the wearer or the caregiver upon use, and thus its properties most associated with the quality and function of the article. By quality, what may be desired is an undergarment like appearance, and pleasant tactile sense such as softness and cushiony touch. Undergarment like appearance may be enhanced by providing the garment-facing surface with certain aesthetic sense such that the belt region and crotch region are not easily distinguished. By function, what may be desired are secure wearability, breathability and comfort, or intuitive signals of such attributes.

Meanwhile, from a manufacturer's point of view, there is desire to provide a high quality absorbent article while controlling cost for making the article; by selecting materials and assembling them in a manner that may provide the best user experience per cost of material.

Based on the foregoing, there is a need for a wearable article providing improved stretchability for ease of application, improved fit for preventing sagging, improved comfort and softness, and improved breathability for skin health. There is also a need for a wearable article having improved aesthetics which intuitively communicates the functional benefits described above. There is also a need for providing such a wearable article which can be economically made.

WO2016101197 relates to a wearable article comprising a main body and a ring-like elastic belt with a front belt and a back belt, each having an inner sheet, an outer sheet, and a plurality of elastic bodies sandwiched therebetween. At least a portion of the elasticity is removed. The length of the article along the longitudinal axis is from 300 mm to 440 mm, and the maximum elastic cut window is less than 100 mm.

US201723183 relates to a fluid acceleration pipe which includes a main body having a pipe cavity and defining a central axis.

WO2016029373 is concerned with a wearable article comprising: a main body with an outer cover layer and a backsheet; and a ring-like elastic belt. The backsheet comprises printing for providing a main artwork existing in the vicinity of the proximal edge of a central panel. The main artwork comprising a belt area artwork displayed on the central panel and a crotch area artwork displayed on the crotch panel.

US2018289563 discloses a wearable article comprising a front region, a back region, a crotch region, and a main body. At least one of the front and back regions comprises a first graphic provided by a first material, and a second graphic provided by a second material. WO2017212857 is said to regard the prevention of collapsing of holes and creasing of both side parts when a perforated nonwoven fabric used as a cover nonwoven fabric, in a two-part exterior shell-type pants-type disposable diaper. The cover nonwoven fabric is provided with a large number of front-rear through-holes with intervals therebetween.

JP3217273 relates to an absorbent article with a front appearance graphic, and a back appearance graphic.

WO2016048337 relates to an absorbent article with an absorbent assembly and a waist assembly attached to the absorbent assembly along a front region and a back region. The waist assembly includes an elastic laminate including a non-apertured body-facing layer, an apertured garment-facing layer, and an elastic layer disposed there between.

JP2015128573 discloses an underpants type absorbent article which is said to improve the gas permeability of an outer wrapper body, reducing stuffiness of the inside.

WO2017212856 provides a two-part shell-type pants-type disposable diaper, wherein a visual effect of holes when a perforated non-woven fabric is used as the cover nonwoven fabric is said to be improved.

SUMMARY OF THE INVENTION

The present invention is directed to a wearable article continuous in a longitudinal direction and a transverse direction comprising a front elastic belt, a back elastic belt, a crotch region, a waist opening, and a pair of leg openings; the crotch region extending longitudinally between the front elastic belt and the back elastic belt;

> each of the front elastic belt and the back elastic belt being a laminate comprising an inner sheet, an outer sheet, and an elastic member running in the transverse direction, and the front elastic belt and the back elastic belt being discontinuous with each other in the crotch region;
> the front elastic belt and back elastic belt having a transverse dimension of LW, the smaller longitudinal dimension of the front elastic belt or the back elastic belt having a dimension of LS, wherein from about 10% to about 40% of LW, and from about 15% to about 75% of LS, preferably from about 30% to about 70% of LS, is removed of its elastic activity, wherein the region of the front elastic belt and the back elastic belt removed of its elastic activity is a non-elastic region 221; the front and back non-elastic regions 221 have a First Belt Pattern FBP and the crotch region has a First Crotch Pattern FCP.

In one aspect, the First Belt Pattern FBP and the First Crotch Pattern FCP are both a Geometric Pattern, and the First Belt Pattern FBP and the First Crotch Pattern FCP both have a Longitudinal Orientation, according to the measurements herein.

In another aspect, both the First Belt Pattern FBP and the First Crotch Pattern FCP form a High Order Pattern HOP having an area dimension of from about 16mm$^2$ to about 200mm$^2$, wherein the First Belt Pattern FBP and the First Crotch Pattern FCP are Geometric Patterns having a common element, and both the First Belt Pattern FBP and the First Crotch Pattern FCP have a Non-directional Orientation, according to the measurements herein.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

> Figure 1A is a perspective view of one embodiment of a wearable article of the present invention.
> Figure 1B is a schematic view of one embodiment of a wearable article of the present invention in a contracted state showing the front side of the article.
> Figure 2A is a schematic plan view of one embodiment of a wearable article of the present invention with the seams unjoined and in a flat uncontracted condition showing the garment facing surface.
> Figure 2B is a schematic cross section view of Figure 2A taken along LX.
> Figure 3A is a schematic plan view of the embodiment of Figure 2A showing the elastic member positioning, elastic bondings and vertical bondings.
> Figure 3B is an expanded schematic plan view of Figure 3A.
> Figure 4A is a schematic cross section view of Figure 3B taken along section line 4-4 of Figure 3B.
> Figure 4B is a schematic cross section view of 4A in a contracted state.
> Figure 5A is a schematic plan view of the elastic belt of the present invention.
> Figure 5B is a plan view of the elastic belt of the present invention in a stretched state.
> Figure 5C is a plan view of the elastic belt of Figure 5B in a contracted state.
> Figure 6A is a schematic plan view of one embodiment of a wearable article of the present invention in a contracted state.
> Figure 6B is a schematic plan view of another embodiment of a wearable article of the present invention in a contracted state.
> Figure 7 is a schematic plan view of another embodiment of a wearable article of the present invention in a contracted state.
> Figure 8 is a schematic plan view of another embodiment of a wearable article of the present invention in a contracted state.
> Figure 9A is a schematic plan view of another embodiment of a wearable article of the present invention in a contracted state.

Figure 9B is a schematic plan view of an embodiment of a pattern of the present invention.

Figure 9C is a schematic plan view of another embodiment of a pattern of the present invention.

Figure 9D is a schematic plan view of another embodiment of a pattern of the present invention.

Figure 10 is a schematic view of an example of a hanger-type sample holding fixture according to the "Whole Article Force Measurement".

Figures 11A-11D are related to the Longitudinal Orientation measurement herein.

Figures 12A - 12C are images of Example 1 FBP in its original and processed forms related to the "Longitudinal/Transverse Distribution" measurements herein.

Figures 13A - 13C are images of Example 1 FCP in its original and processed forms related to the "Longitudinal/Transverse Distribution" measurements herein.

Figures 14A - 14C are images of Example 2 FBP in its original and processed forms related to the "Longitudinal/Transverse Distribution" measurements herein.

Figures 15A - 15C are images of Example 2 FCP in its original and processed forms related to the "Longitudinal/Transverse Distribution" measurements herein.

Figures 16A - 16H are synthetic photographs of pant type articles utilized as visual presentations in the Examples section.

## DEFINITIONS

As used herein, the following terms shall have the meaning specified thereafter:

"Wearable article" refers to articles of wear which may be in the form of pants, taped diapers, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.

"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Proximal" and "distal" refer respectively to the position closer or farther relative to the longitudinal center of the article.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Dimension", "Length", "Width", "Pitch", "Diameter", "Aspect Ratio", "Angle", and "Area" of the article are all measured in a state wherein the article is extended to the Full Stretch Circumference W1 according to the "Whole Article Force Measurement" herein, and utilizing a ruler or a loupe, unless specified otherwise.

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to $0 \pm 2$, and a b* value equal to $0 \pm 2$ according to the CIE L* a* b* color system.

## DETAILED DESCRIPTION OF THE INVENTION

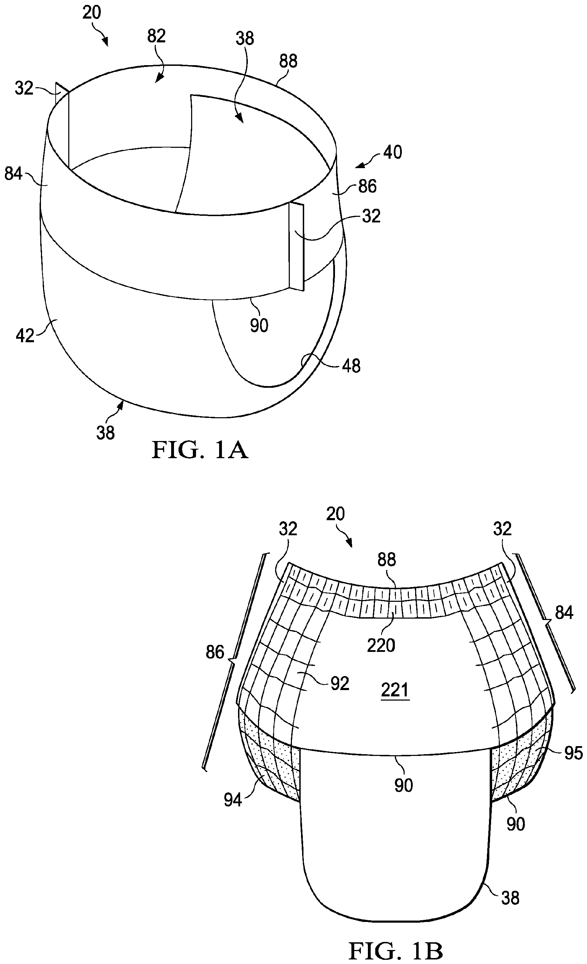

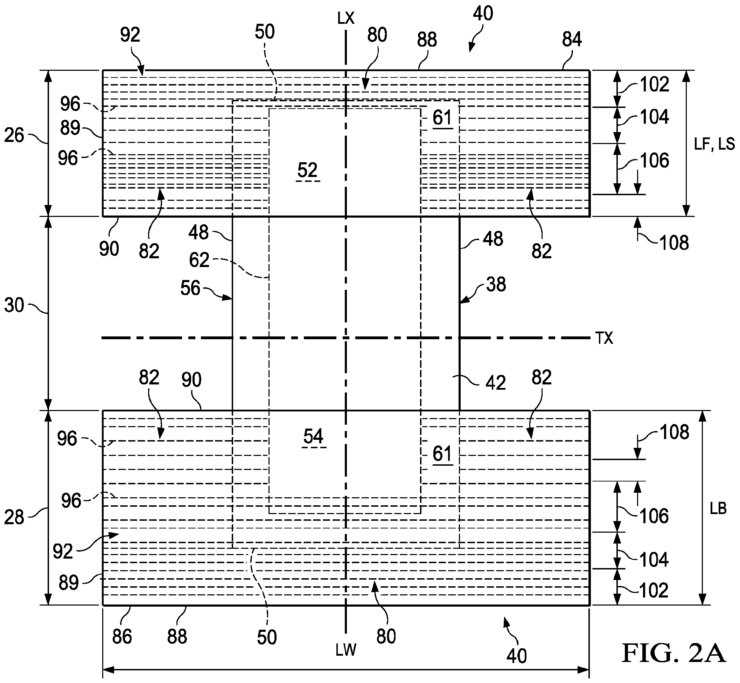

Figure 1A is a perspective view of a wearable article (20) of the present invention, Figure 1B is a schematic view of a wearable article of the present invention in a contracted state showing the front side, and Figure 2A is a schematic plan view of a wearable article with the seams unjoined and in its flat uncontracted condition showing the garment-facing surface. The wearable article (20) has a longitudinal centerline LX which also serves as the longitudinal axis, and a transverse centerline TX which also serves as the transverse axis. The wearable article (20) has a body facing surface, a garment facing surface, a front elastic belt (84), a back elastic belt (86), a crotch region (30), and side seams (32) which join the front elastic belt (84) and the back elastic belt (86), to form two leg openings and a waist opening.

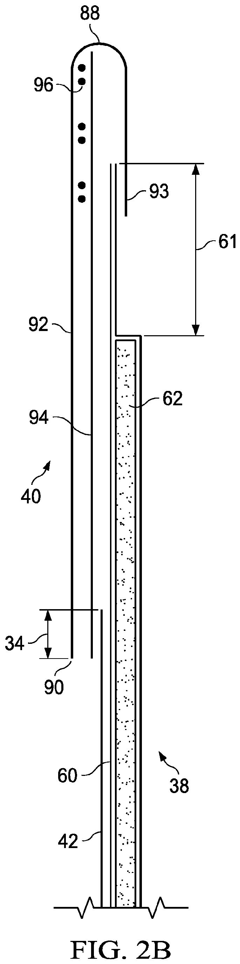

The wearable article (20) may be a belt-type pant as in Figures 1A, 1B, 2A and 2B comprising a central chassis 38 to cover the crotch region (30) of the wearer, a front elastic belt (84) and a back elastic belt (86) (hereinafter may be referred to as "front and back elastic belts"), the front and back elastic belts (84, 86) forming a discrete ring-like elastic belt (40) extending transversely defining the waist opening. For the belt-type pant, the discrete ring-like elastic belt (40) may also be referred to as the elastic belt (40). For the belt-type pant as in Figures 1A, 1B, and 2A, the front and back elastic belts (84, 86) and the central chassis (38) jointly define the leg openings. For the belt-type pant, the front elastic belt 84 is the front region (26), and the back elastic belt (86) is the back region (28), and the remainder is the crotch region (30). While not shown, the wearable article (20) may be a uni-body type pant configured such that the outer cover of the central chassis (38) and the elastic belt (40) are common. For the uni-body type pant, the portion extending in the transverse direction between the side seams (32), respectively, are considered the front region (26) and the back region (28), and the remainder is the crotch region (30). For the uni-body type pant, the front region (26) is considered the front elastic belt (84), and the back region (28) is considered the back elastic belt (86).

The central chassis (38) may comprise a topsheet, a backsheet and an absorbent core (62) disposed between the topsheet and the backsheet, and further an outer cover layer (42) for covering the garment-facing side of the backsheet. The topsheet may be a water permeable substrate. The backsheet may be a water impermeable film. The outer cover layer (42) may be a nonwoven sheet. The central chassis (38) may contain an absorbent core (62) for absorbing and containing body exudates disposed on the central chassis (38), and an absorbent material non-existing region (61) surrounding the periphery of the absorbent core (62). The absorbent material non-existing region (61) may be made of the topsheet and/or the backsheet and/or the outer cover layer (42) and/or other parts configuring the central chassis (38). In the embodiment shown in Figure 2A, the central chassis (38) has a generally rectangular shape, left and right longitudinally extending side edges (48) and front and back transversely extending end edges (50). The absorbent core (62) may exist through the entire longitudinal dimension of the crotch region and extending at least partly in the front region (26); or at least partly in both the front and back regions (26, 28). The central chassis (38) may have a front waist panel (52) positioned in the front region (26) of the absorbent article (20), a back waist panel (54) positioned in the back region (28), and a crotch panel (56) between the front and back waist panels (52, 54) in the crotch region (30). The center of the front elastic belt (84) is joined to a front waist panel (52) of the central chassis (38), the center of the back elastic belt (86) is joined to a back waist panel (54) of the central chassis (38), the front and back elastic belts (84, 86) each having a left side panel and a right side panel (82) where the central chassis (38) does not overlap. The central chassis has a crotch panel (56) positioned between the front waist panel (52) and the back waist panel (54).

The absorbent core (62) may include an absorbent layer and an acquisition layer. The absorbent layer is the region wherein absorbent materials having a high retention capacity, such as superabsorbent polymers, are present. The absorbent layer may be substantially cellulose free. Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multiconstituent fibers such as side by side, core/sheath or island in the sea type fibers. Such synthetic fibers may be formed via a spunbonding process or a meltblowing process. The acquisition layer facilitates the acquisition and the distribution of body exudates and may be placed between the topsheet and the absorbent layer. The acquisition layer may include cellulosic fibers.

The absorbent layers may be disposed in plurality in the absorbent core (62). Some portions of the absorbent layers may be configured to have substantially no absorbent material to form a channel or a plurality of channels. Channels may be useful for allowing the absorbent core (62) to bend upon swelling with fluids, such that the absorbent article conforms to the wearer's body after swelling and prevent sagging of the article. The channels may also be formed in the acquisition layer, and may be configured to at least partly match the channels of the absorbent layer in the thickness direction.

The elastic belt (40) of the article of the present invention acts to dynamically create fitment forces and to distribute the forces dynamically generated during wear. The front and back elastic belts (84, 86) may be joined with each other only at the side edges (89) to form side seams (32), a waist opening and two leg openings. Each leg opening may be provided with elasticity around the perimeter of the leg opening. The elasticity around the leg opening may be provided by the combination of elasticity from the front elastic belt (84), the back elastic belt (86), and the central chassis (38).

Referring to Figure 2B, the longitudinal length of the backsheet and the outer cover layer (42) may be the same, or may be varied. For example, the outer cover layer (42) may have a shorter length compared to that of the backsheet, such that the outer cover layer (42) is devoid where the central chassis (38) overlaps the elastic belt (40). By such configuration, the elastic belt may have better breathability. Further, such configuration may provide cost saving. Further, such configuration may prevent interference of patterns provided on the non-elastic region (221) of the elastic belt (40) and the crotch region (30), as further detailed below. The transverse width of the backsheet and the outer cover layer (42) may be the same, or may be varied. For example, the backsheet may have a shorter transverse width compared to that of the outer cover layer (42). By such configuration, the longitudinal side edges (48) of the crotch panel (56), which make part of the leg openings, may have better breathability. Further, such configuration may provide cost saving.

The front elastic belt (84) and back elastic belt (86) are configured to impart elasticity to the belt (40). Referring to Figures 1B 2A, and 2B, the front elastic belt (84) and the back elastic belt (86) may each be formed by a laminate comprising a plurality of elastic bodies (96) running in the transverse direction, an inner sheet (94), an outer sheet (92), and an outer sheet fold over (93) wherein the outer sheet fold over is an extension of the outer sheet material formed by folding the outer sheet material at the distal edge (88) of the front and back elastic belts; wherein the belt elastic bodies (96) are sandwiched between two of these sheets. The front elastic belt (84) and the back elastic belt (86) may each be made only by elastic bodies (96), the inner sheet (94), the outer sheet (92), and the outer sheet fold over (93). The various patterns, discussed below, may be provided on the outer sheet (92). The belt elastic bodies (96) may extend in the transverse direction to provide a ring like elastic belt (40) when the front elastic belt (84) and the back elastic belt (86) are joined. At least some of the elastic bodies (96) extend in the transverse direction substantially parallel to each other. All of the elastic bodies (96) may extend in the transverse direction substantially parallel to each other. Such an article may be economically made. The front and back elastic belt (84, 86) each may have transversely continuous proximal and distal edges, the proximal edge (90) being located closer than the distal edge (88) relative to the longitudinal center of the article. The elastic bodies (96) may be disposed in the same or different denier, interval, and force between the front and back elastic belts (84, 86), as well as in different longitudinal positions of the belt.

Referring to Figure 2A, the transverse width LW of the back elastic belt (86) in the uncontracted condition is the same as the transverse width of the front elastic belt (84) of the same condition. Along its entire width LW, the back elastic belt (86) between the back distal edge (88) and the back proximal edge (90) has a longitudinal dimension LB, and the front elastic belt (84) between the front distal edge (88) and the front proximal edge (90) has a longitudinal dimension LF. LB and LF may be the same or different. The shorter of LB and LF is defined as LS.

Referring to Figure 2A, LB may be greater than LF. In such configuration, when the wearable article is assembled to form the waist opening and the leg openings, the wearable article (20) is folded along the transverse centerline TX such that the front distal edge (88) is aligned with the back distal edge (88). The front side edge (89) is also aligned with a portion of the back side edge (89). Then the front elastic belt (84) and the back elastic belt (86) are joined at the front and back side edges (89) at the seams (32). The front and back proximal edges (90), however, may not be aligned to one another. The back proximal edge (90) may be disposed longitudinally closer than the front proximal edge (90) relative to the transverse center line TX such that the proximal portion of the back side panel (82) extends toward the crotch panel (56) of the central chassis (38) beyond the front proximal edge (90). The side edge of the proximal portion of the back side panel (82) may not be joined to anywhere and free from attachment. Thus, the proximal portion of the back side panel (82) provides a buttock cover (95), as in Figure 1B.

Referring to Figure 1B and 2A, the front and back elastic belts (84, 86) are treated such that certain regions are removed of its elastic activity to form a non-elastic region (221). For each of the front and back elastic belt (84, 86), from about 10% to about 40% of LW, and from about 15% to about 75% of LS, or from about 30% to about 70% of LS, is removed of its elastic activity. The non-elastic region (221) may overlap the front and/or back waist panel (52, 54) of the central chassis (38). Removal of elasticity from a certain area superposing the front and/or back waist panel (52, 54) may be advantageous when the central chassis (38) comprises an absorbent core (62), in that elasticity in the front and/or back area overlapping the absorbent core (62) may cause bunching of the absorbent layer or any of the layers in the absorbent core (62) and interfere with close fit of the central chassis (38) to the wearer.

The remainder of the front elastic belt (84) and the back elastic belt (86) each comprise an elastic region, the elastic regions at least extending along the waist opening in the transverse direction to provide a circumferential elastic region. The circumferential elastic region along the waist opening defines the Upper Gather Region (220).

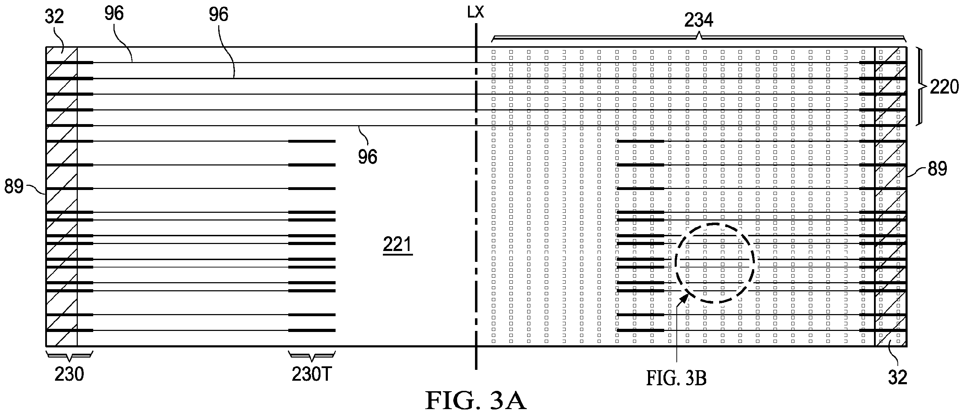

Referring to Figure 3A, the laminate may be made by bonding the elastic members (96) to at least one of the inner sheet (94) and the outer sheet (92), via a combination of an elastic bonding (230) and a vertical bonding (234). In Figure 3A, the front elastic belt (84) is shown with the elastic members (96) and elastic bonding (230) expressed in solid lines. In Figure 3A, the vertical bonding (234) is only expressed in the right side of the front elastic belt (84), and the side seams (32) are shown in an unjoined state.

What is meant by elastic bonding (230) herein is a bonding that bonds the elastic member (96) along the side edges (89) of the front and back elastic belts (84, 86). The elastic bonding (230) may be continuously applied to each elastic member (96) for a length of at least about 10mm, or from about 10mm to about 60 mm in the direction of stretch adjacent the side edges (89) of the front and back elastic belts (84, 86), including the length planned for side seaming. The elastic bonding (230) is to provide relatively strong bonding for the elastic member (96) and thus securely anchor the elastic member (96) within the laminate. The anchoring may be assisted by the side seaming. A certain percentage, or a greater percentage, of the dimension of the elastic bonding (230) along the side edges (89) may be seamed. The elastic bonding may also be utilized for an effective process of deactivating a limited transverse dimension of the elastic member (96). Referring to Figures 2A and 3A, the elastic member (96) may be deactivated in portions overlapping the absorbent core (62). In addition to the side edge regions, the elastic bonding (230T) may be provided on both sides of the certain transverse dimension of the elastic member (96) which is planned to be deactivated, wherein the portion of the elastic member between the elastic bondings (230T) are severed and deactivated. The deactivated portions of the elastic member is not shown in the Figures. Such deactivation may be referred to herein as tummy cut, and the deactivated region may match the non-elastic region (221).

What is meant by vertical bonding (234) herein is a bonding applied to at least one of the inner sheet (94) and the outer sheet (92) with intervals in the transverse direction for intermittently bonding the inner sheet (94) and the outer sheet (92). The vertical bonding (234) may also bond the elastic member (96) to at least one of the inner sheet (94) and the outer sheet (92). The vertical bonding (234) may only be provided to the outer sheet (92). Referring to Figure 3A, vertical bonding (234) may be provided in a pattern for the entire area of the laminate. By providing vertical bonding (234) in a pattern for the entire area of the laminate, the vertical bonding (234) may serve as a bonding for the inner and outer sheets (92, 94) in regions where the elastic members (96) are severed. Vertical bonding (234) may be provided in regions adjacent the side edges (89) and thus overlapping regions where the elastic bondings (230) are provided. Alternatively, vertical bonding (234) may be provided only in regions where the elastic bondings (230) are not provided. Vertical bonding (234) may be provided at least in regions where the elastic member (96) is in active elasticity, wherein the elastic bondings (230) are devoid.

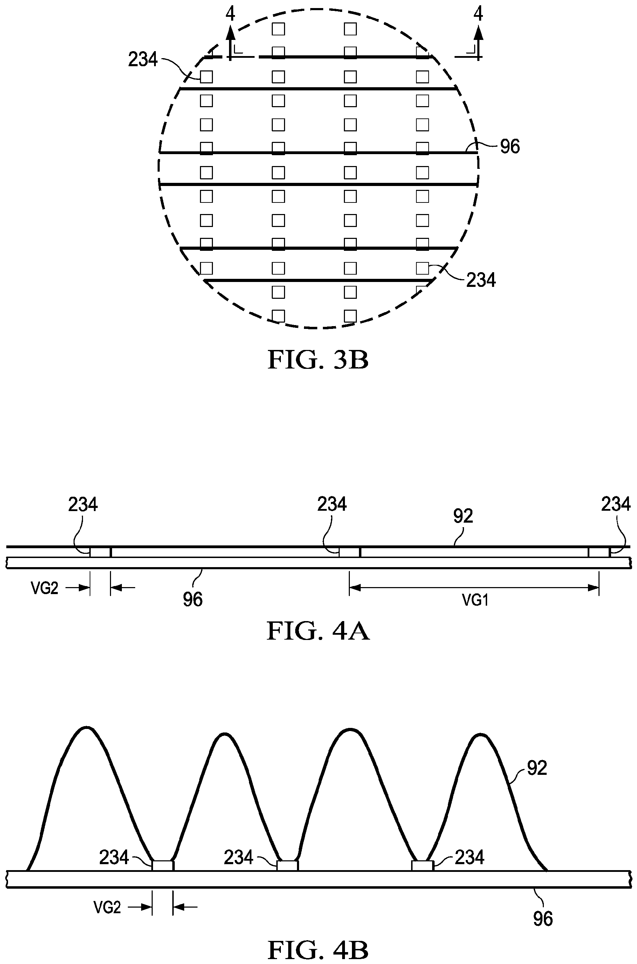

Referring to Figure 4A, the vertical bonding (234) is observed in the thickness direction of the laminate along a single elastic member (96) in an extended state in the transverse direction, wherein Figure 4A only expresses the outer sheet (92), vertical bonding (234), and the elastic member (96), wherein the vertical bonding (234) is provided on the outer sheet (92). The vertical bonding (234) may have a transverse dimension VG2 and provided as a continuous pattern aligned in the longitudinal direction, each longitudinal pattern of vertical bonding (234) spaced apart from each other with a transverse pitch VG1, wherein VG1 may be from about 2mm to about 15 mm, and VG2 may be from about 0.2 mm to about 7 mm. When focusing on one elastic member (96), the vertical bonding (234) may provide intermittent bonding between the elastic member (96) and one of the inner sheet (94) and the outer sheet (92), or between the elastic member (96) and the outer sheet (92). This is in contrast with the elastic bonding (230) which is provided continuously along a certain length of the elastic member (96) in the direction of stretch. As such, in the region where the elastic member (96) is only intermittently bonded to one of the inner sheet (94) and the outer sheet (92), the portion of the elastic member (96) between the vertical bonding (234) in the transverse direction is unattached to any other part of the laminate. In Figure 4A, the elastic member (96) is bonded to the outer sheet (92). Referring to Figure 4B, when the elastic member (96) is allowed to contract, this causes the unattached portion of the outer sheet (92) to fold away from the elastic member (96) and form gathers. As such, compared to areas where the elastic bonding (230) is applied, the outer sheet (92) has less restriction in creating gathers.

Without being bound by theory, it is believed that by having less restriction for the inner sheet (94) and outer sheet (92) against the elastic members (96), this contributes in creating gathers of improved regularity, in that a significant amount of the inner and outer sheet materials (92, 94) existing between the vertical bondings (234) are available for creating gathers continuous in the longitudinal direction. Without being bound by theory, it is also believed that, by having less restriction for the inner and outer sheet materials (92, 94) against the elastic members (96), this allows improved stretchability of the elastic members (96), which may provide ease of application. Compared to elastic belts made only by elastic bonding (230) wherein all of the elastic members (96) are continuously bonded, the elastic belt (40) of the present invention may have a lower Stretch Circumference Force, according to the measurements herein. Further, despite such relatively low Stretch Circumference Force, the elastic belt (40) of the present invention may maintain a suitable Fit Circumference Force, according to the measurements herein. Without being bound by theory, it is also believed that, by having less restriction for the inner and outer sheet materials (92, 94) against the elastic members (96), this improves the breathability of the overall laminate, which may enhance skin health. Without being bound by theory, it is also believed that vertical bonding (234) provides a configuration wherein a greater percentage of the inner and outer sheet materials (92, 94) are available for forming the outer surfaces of the laminate when the elastic belt (40) is contracted, while the elastic members (96) remain positioned inside the thickness of the laminate. As such, the laminate is provided with improved loft and thickness, thus imparting improved comfort and softness when worn. Further, without being bound by theory, in that there is a great percentage of the inner and outer sheet materials (92, 94) available for forming the outer surfaces of the laminate in high regularity when the elastic belt (40) is contracted, this provides the body facing surface of the elastic belt (40) to have higher stiffness in the longitudinal direction, thus contributing in improved fit for preventing sagging. Still further, in that the elastic members are less visible when the elastic belt (40) is contracted, this further enhances the aesthetically pleasing regularity of gathers.

In order to make available for gathering a significant amount of the inner and outer sheet materials (92, 94) between the vertical bondings (234) in the transverse direction, VG1 may be from about 2 times to about 20 times, or from about 3.5 times to about 10 times of VG2.

By bonding, what may be utilized are any methods known in the art, such as use of hot melt adhesive, thermal energy, and ultrasonic energy. Bonding strength may be adjusted by the area of bonding, or by different adhesion or energy level provided by the bonding, for example, adjusting the amount and strength of an adhesive agent. The bonding strength of the elastic bonding (230) and the vertical bonding (234) may be the same or may be varied. The elastic bonding (230) and the vertical bonding (234) may be provided by the same hot melt adhesive.

The vertical bonding (234) may be a continuous line extending in the longitudinal direction. Referring to Figure 3B, the vertical bonding (234) may be an array of discrete bondings aligned in the longitudinal direction. Each discrete vertical bonding (234) may have a longitudinal dimension of from about 0.5mm to about 10mm, and a longitudinal pitch of from about 1mm to about 10mm, or from about 0.8mm to about 5mm. By providing the vertical bonding (234) in an array of discrete bondings, the overall area of bonding may be decreased. This is advantageous for maintaining the laminate in a soft tactile sense, in that bonding may provide the inner and outer sheet material (92, 94) stiffer. Further, this may save material or energy for the bonding. Each discrete vertical bonding (234) may be provided in appropriate longitudinal pitch such that there is at least one discrete bonding that bonds each elastic member (96), however, this is not essential. Rather, it is essential that there is at least one discrete vertical bonding (234) existing in each longitudinal spacing of the elastic members (96), such that the neighboring elastic members (96) do not contact each other. In that the elastic bonding (230) provides secure bonding of the elastic member (96) along the side seams, as well as the outer periphery of the non-elastic region (221), so long as there is at least one discrete vertical bonding (234) existing in each longitudinal spacing of elastic members (96), this prevents the elastic member (96) from moving away from its intended position. For an entire front elastic belt (84) or an entire back elastic belt (86) there may be no elastic member (96) bonded to the inner sheet (94) or the outer sheet (92) by a discrete vertical bonding (234). For an entire front elastic belt (84) or an entire back elastic belt (86), at least one to about 50% of the elastic members (96) may be bonded to the inner sheet (94) or the outer sheet (92) by the discrete vertical bonding (234). For an individual elastic member (96) along its activated length, some portions may be bonded by the discrete vertical bonding (234), while some portions may be left unbonded by the discrete vertical bonding (234).

Referring to Figure 2A, for the belt-type pant, the proximal edges (90) of the front and back elastic belt (84, 86) may be provided with an end seal in order to keep the inner and outer sheets (92, 94) closed at the proximal edges (90) and thus prevent elastic members (96) from being accessible. Such unaccessibility of elastic members (96) may be particularly advantageous when the article is for a young wearer. Alternatively or additionally, the elastic member (96) which is positioned closest to the proximal edge (90) may be provided with a bonding along the transverse dimension of the elastic member (96) in state of active elasticity.

Referring to Figure 2A, the elastic member (96) may be made by a plurality of elastic strands (96) running parallel to each other in the transverse direction, wherein the laminate has at least a region wherein the elastic strands (96) have a longitudinal pitch of from about 2mm to about 20mm, or from about 3mm to about 12mm, or from about 3mm to about 7mm. At least some of the Upper Gather Region (220) may have the elastic strands (96) disposed in a longitudinal pitch of from about 3mm to about 7mm. Without being bound by theory, it is believed that such longitudinal pitch of the elastic strands (96), combined with the transverse pitch of the vertical bonding (234) as described above, contributes in creating gathers of improved regularity by providing the appropriate longitudinal continuity of material provided by the stiffness of the inner and outer sheet materials (92, 94). At least a portion of the Upper Gather Region (220) may have the elastic strands (96) disposed in a constant longitudinal pitch, the constant pitch being from about 2mm to about 20mm, or from about 3mm to about 12mm, or from about 3mm to about 7mm, with a deviation of no more than about 1.5mm. Without being bound by theory, it is believed that such constant pitch of the elastic strands (96) contributes in creating gathers of improved regularity and continuity.

The front and back elastic belts (84, 86) may be made by running the continuous inner and outer sheet materials as well as the continuous elastic strands along the transverse axis of the article, and bonding them via the elastic bondings (230) and vertical bondings (234). During manufacture, the continuous inner and outer sheet materials and continuous elastic strands may be transferred in the machine direction, wherein the machine direction of manufacture matches the transverse axis TX of the article. In such manufacturing process, the vertical bondings (234) are provided continuous or discretely aligned in the cross machine direction and intermittently spaced apart by a pitch of VG1 in the machine direction of manufacture. The longitudinal pattern of vertical bondings (234) may match the cross machine direction of manufacture, namely the longitudinal axis LX of the article, or may be slightly titled for better control of the process, particularly when the vertical bonding (234) is provided by applying bonding on a rotating roller. The vertical bonding (234) may be tilted with an angle from the cross machine direction of manufacture, namely the longitudinal axis LX of the article, by from about 0.1 to about 30 degrees in either clock-wise or counterclock-wise direction, or from about 0.1 to about 15 degrees in either clock-wise or counterclock-wise direction.

The tensile stress (N/m) of the entirety of the front and back elastic belts (84, 86), respectively, may be profiled in order to provide the functional benefits of the present invention, such as ease of stretch and application, while also maintaining certain force during wear, to prevent the article from sagging after loading. When the elasticity of the front and back elastic belts (84, 86) are provided by a plurality of elastic members (96) running in the transverse direction, the tensile stress may be adjusted by one or more of the following methods; 1) elongation rate of the elastic member (96); 2) density (dtex) of the elastic member (96); 3) longitudinal interval of multiple elastic members (96); and 4) effective length of elasticity of the elastic member (96) in the transverse direction. By elongation, "0% elongation" is meant the original length of the elastic member. When a portion of an elastic member (96) is removed of its elasticity, the remainder of the intact elastic member capable of imparting elasticity is defined as the "effective length of elasticity of an elastic member".

Referring to Figure 2A, the front and back elastic belts (26, 28) may each be divided into 4 zones spanning in the transverse direction and defined of its position from the distal edge (88) to the proximal edge (90) relative to the percentage of the seam length LS. In the example of Figure 2A, the entirety of the length of the belt side edge (89) of the front region (26) is the front elastic belt (84), and is seamed with a certain length of the belt side edge (89) of the back region (28) which is the back elastic belt (86) to define a seam length LS. When seam length LS is considered 0% at the distal edge (88) and 100% at the proximal edge (90) of the side seam (32), the zones are defined as such: 0-25% is the waist zone (102), 25-50% is the distal tummy zone (104), 50-85% is the proximal tummy zone (106), and 85-100% is the leg zone (108). When there is an elastic member disposed at 25% from the distal edge 88, such elastic member is considered to be included in the waist zone (102). When there is an elastic member disposed at 50% from the distal edge (88), or 85% from the distal edge (88), such elastic member is considered to be included in the proximal tummy zone (106).

In the article of the present invention, the tensile stress of the front proximal tummy zone (106) may be provided higher than the tensile stress of any of the front waist zone (102), the front distal tummy zone (104), or the front leg zone (108). The tensile stress of the front proximal tummy zone (106) may be higher than the tensile stress of any other zone, either in the front or the back. The tensile stress of the back distal tummy zone (104) may be provided higher than any of the tensile stress of the back waist zone (102), the back proximal tummy zone (106), or the back leg zone (108). When comparing the 4 zones each of the front elastic belt and the back elastic belt, the tensile stress may be provided greatest in the order of: the front proximal tummy zone (106), followed by the back distal tummy zone (104). Without being bound by theory, such profiling of the tensile stress per zone is believed to provide the article of the present invention with a shaped elastic belt (40) that conforms well to a human body, particularly to a lower torso of a child of less than 36 months of age, and therefore provide good fit and comfort to the wearer, without compromise of sagging prevention or leakage prevention. Namely, the front proximal tummy zone (106) is subject to high tensile stress such that the article may be anchored against the wearer's trochanter, while leaving more area for the back proximal tummy zone (106) to accommodate the wearer's buttock. As long as the article is anchored securely at the trochanter, the Upper Gather Region (220) may be provided in relatively lower tensile stress. Without being bound by theory, it is believed that such relatively lower tensile stress contributes in providing the Upper Gather Region (220) with improved regularity of gathering, as well as soft fit.

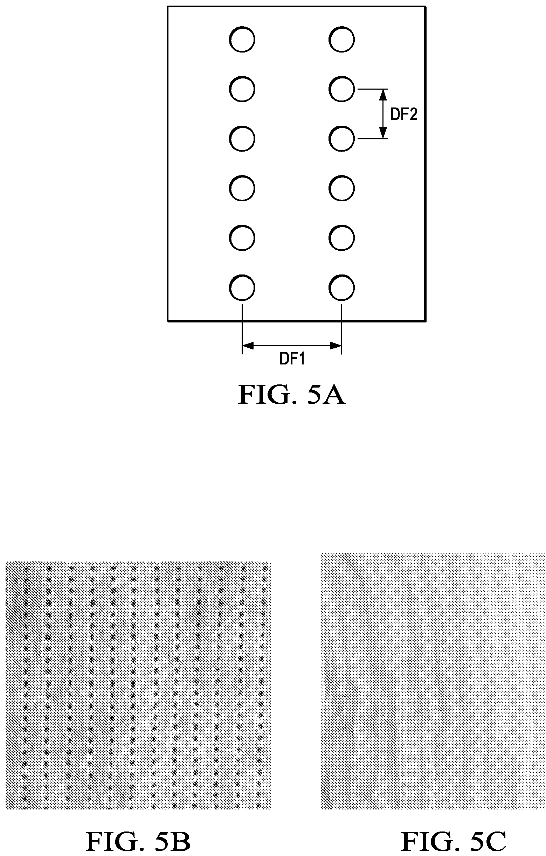

In the present invention, at least one of the inner sheet (92) and the outer sheet (94) may further comprise a plurality of deformations wherein the deformations are aligned in the longitudinal direction. Deformations may be apertures, slits, engravings, embossings, projections, or any other permanent deformation to the nonwoven material for making the inner sheet (92) and/or the outer sheet (94), so long as they are aligned in the longitudinal direction. For example, referring to Figure 5A, deformations on the outer sheet (92) in the form of apertures are expressed. Each longitudinal deformation pattern may be spaced apart from each other with a transverse pitch of DF1, wherein VG1 is greater than DF1, or wherein VG1 is at least about 1.5 times, or at least about 2 times, of DF1. Without being bound by theory, such deformation provided in relationship with the vertical bonding (234) assists the nonwoven material for making the inner sheet (92) and/or the outer sheet (94) to be folded within dimension VG1, the folding being continuous in the longitudinal direction. As such, the regularity of gathering is enhanced. The longitudinal deformation pattern may or may not be registered with the longitudinal pattern of the vertical bonding (234). In fact, it is the finding of the present invention that, even when the longitudinal deformation pattern is not registered with the longitudinal pattern of the vertical bonding (234), this still enhances the regularity of gathering. In that registration of the longitudinal deformation pattern and the longitudinal pattern of the vertical bonding (234) may require precision of process, such registration may be omitted. Even when the longitudinal deformation pattern is not registered with the longitudinal pattern of the vertical bonding (234), by providing DF1 and VG1 in a relationship other than multiple number of integers, a majority of the longitudinal deformation patterns fit within the longitudinal patterns of the vertical bonding (234) and assists gather forming.

The deformation may be a continuous line extending in the longitudinal direction, or an array of discrete deformations aligned in the longitudinal direction and spaced apart from each other with a longitudinal pitch of DF2, wherein DF2 is not greater than DF1, as in Figure 5A. By providing the deformations in an array of discrete deformations, the overall area weakened or stiffened by the deformation may be decreased. By providing DF2 the same or smaller than DF1, this facilitates the folding of the inner and outer sheet material (92, 94) in the longitudinal direction as described above.

The deformation may be an aperture on the outer sheet, wherein the aperture is in the shape of a circle, oval, or polyhedron, and having have a minor radius of at least about 0.1mm, or from about 0.1mm to about 1.5, or from about 0.4mm to about 1.5mm, and having an aspect ratio of less than about 3, or less than about 2. What is meant by minor radius herein is the radius of a circle, minor radius of an oval, or one half the shortest dimension of a polyhedron. Apertures of such size and shape may be visible to the naked eye on the garment-facing surface, and thus connote breathability and high quality of the gather, as well as the entire laminate. Thus, apertures may be provided on the outer sheet (92). The apertures may be provided on both the inner and outer sheets (92, 94) for enhancing breathability. Further, by providing VG1 greater than DF1, the apertures are positioned on the folding as described above, thus the visibility of the apertures are enhanced, even when the gathers are in contracted state. Figure 5B is a plan view of the elastic belt of the present invention in a stretched state, whereas Figure 5C is the same elastic belt in a contracted state. In the elastic belt of Figures 5B-5C, VG1 is about 1.5 times that of DF1. As can be seen in Figure 5C, by providing the VG1 and DF1 relationship as such, at least one longitudinal row of apertures are continuously folded in the longitudinal manner to provide a longitudinal continuous gather, while the apertures are situated nearby the mountain of each gather. As such, visibility of the apertures are enhanced. This may further enhance breathability, as well as the breathability perception.

The front and back non-elastic regions (221) are removed of elasticity and have less or no gathering, thus the material forming the garment facing side of the non-elastic regions (221) have high visibility. Further, the front and back non-elastic regions (221) are adjacent the Upper Gather Region (220) as well as the crotch region (30), and positioned more or less in the longitudinal and transverse center of the article when worn. Thus, the aesthetic effect of the non-elastic regions (221) in coordination with the remainder of the article provide certain perception of quality for the entire article. The aesthetic effect of the non-elastic regions (221) may be coordinated with the Upper Gather Region (220) to enhance the directionality of the longitudinally continuing gathers of the Upper Gather Region (220). Additionally or alternatively, the aesthetic effect of the non-elastic regions (221) may be coordinated with the crotch region (30) to enhance the integral undergarment like appearance of the article. Such aesthetic effects are expected to provide the perception of a high quality article.

The non-elastic regions (221) and crotch region (30) are provided with an Aesthetic Pattern or a Geometric Pattern. In the present invention, by Aesthetic Pattern, what is meant is a repeating visual presentation which may be a Geometric Pattern, or a printed pattern, so long as the repeating visual presentation is visible from the garment-facing side by the naked eye. In the present invention, by Geometric Pattern, what is meant is a repeating visual presentation which is a permanent deformation to the material forming the garment-facing side of the non-elastic region (221), and which is visible from the garment-facing side by the naked eye. Permanent deformation may be apertures, embossings, and other textural deformations provided on the garment facing surface of the article. The garment facing surface of the crotch region (30) may be the outer cover layer (42). Permanent deformations that are not visible to the naked eye are not considered as a Geometric Pattern herein. Permanent deformations not visible to the naked eye include those that are too fine or too subtle for the naked eye to perceive, those which are provided in such uniformity that the deformation may not be perceived, and those which are not disposed on the garment facing surface. Permanent deformations may be the same or different deformations as described above for assisting gather forming. Printed patterns may be those directly printed by color on the garment facing surface of the non-elastic region (221) or crotch region (30), and/or printed on layers superposing the non-elastic region (221) or crotch region (30) and visible through the layers overlapping them. For example, the printed pattern may be provided on the garment facing surface of the inner sheet (94). For example, the printed pattern may be provided on a layer of the central chassis overlapping with the non-elastic region (221) or crotch region (30).

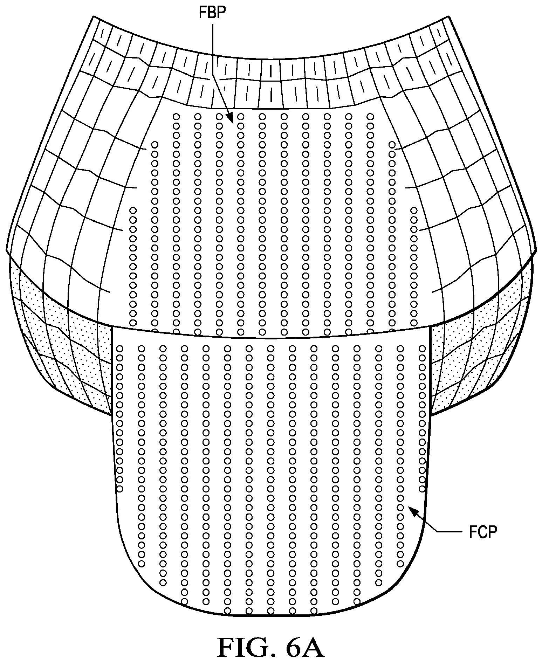

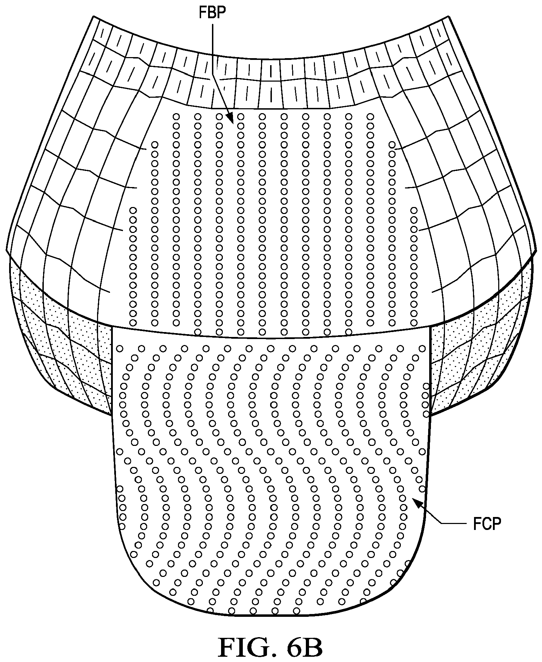

The non-elastic region (221) herein is provided with a First Belt Pattern FBP. The First Belt Pattern FBP may have a Longitudinal Orientation, according to the measurements herein. Longitudinal Orientation may be measured by Observation. Alternatively or additionally, Longitudinal Orientation may be determined by Image Analysis wherein the First Belt Pattern FBP may have a Longitudinal Distribution of at least about 35%, or at least about 50%, or at least about 70%, and a Transverse Distribution of no more than about 35%, or no more than about 20%, or no more than about 10%, according to the measurements herein. What is described by the Longitudinal Orientation herein, is the longitudinally directed impression that the naked eye perceives when observing the repeating visual presentation of a pattern. For example, referring to Figures 6A and 6B, the article of the present invention is shown in a contracted state observed from the front side. The non-elastic region (221) of the front elastic belt is provided with a First Belt Pattern FBP of apertures which are visible from the garment facing side. The apertures are aligned in a more or less straight line extending in the longitudinal direction, and each of such lines are repeated in the transverse direction with spacing. The First Belt Pattern FBP of Figures 6A and 6B have a Longitudinal Orientation.

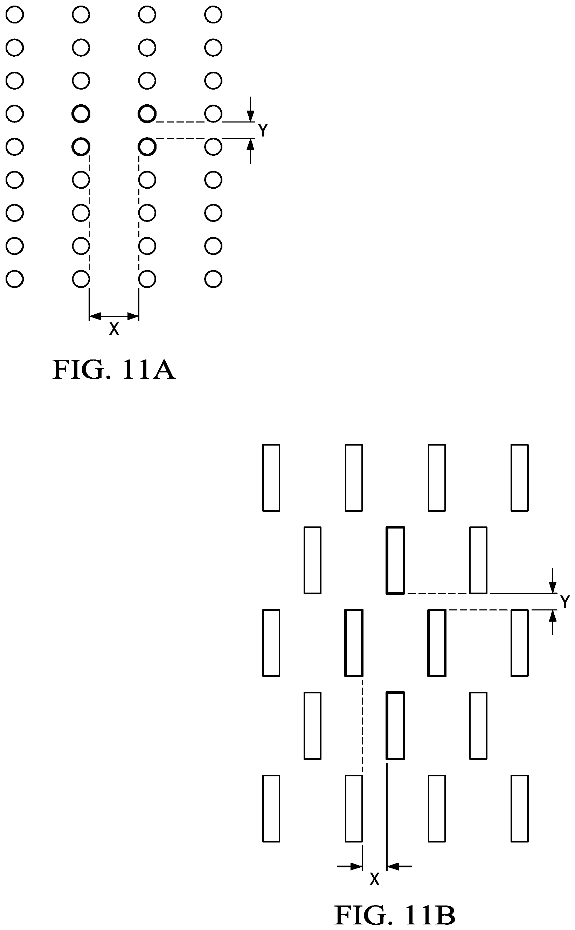

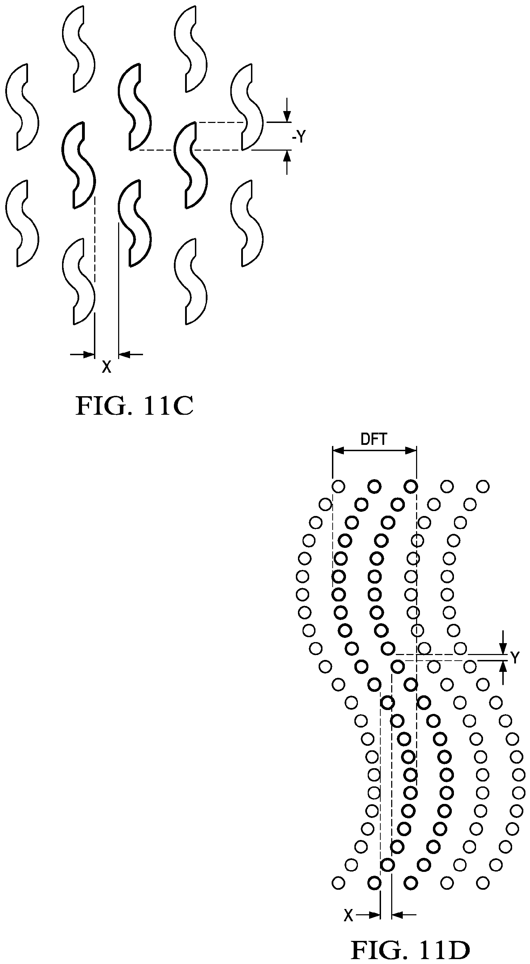

Referring to Figures 11A - 11D, what specifically is meant by Longitudinal Orientation measured by Observation is explained. First, a Basic Unit which is a minimum complete unit of the deformations or printings that repeats to create the pattern is identified. The pattern may be a regularly and continuously repeating pattern of deformation or printing in both the longitudinal and transverse directions having consistent spacing, with no specific boundary of the pattern. Such regularly and continuously repeating patterns having consistent spacing are referred to as randomly repeating patterns. The Basic Unit of a randomly repeating pattern is defined as a group of at least 3, or at least 4, individual deformations or printings, which is capable of surrounding a specific area, which is defined as a Basic Unit Area. For example, Figure 11A is a randomly repeating pattern having a Basic Unit consisting of 4 deformations, each deformation having a circle shape which may be apertures, wherein the Basic Unit Area is an approximate rectangle. In another example, Figure 11B is a randomly repeating pattern having a Basic Unit consisting of 4 deformations, each deformation having a bar shape which may be an embossing, wherein the Basic Unit Area is an approximate diamond. In yet another example, Figure 11C is a randomly repeating pattern having a Basic Unit consisting of 4 deformations, each deformation having an S shape which may be a slit, wherein the Basic Unit Area is an approximate diamond. In yet another example, Figure 11D is a randomly repeating pattern having a Basic Unit consisting of about 50 deformations lined up in 2 sinusoidal waves, each deformation having a circle shape which may be apertures, wherein the Basic Unit Area is an approximate wave having a substantially constant transverse dimension. Within the Basic Unit, the closest adjacent deformation or printing in the longitudinal direction and the transverse direction are identified, and their spacings measured. Those patterns having the X spacing longer than the Y spacing by at least 10% are considered having a Longitudinal Orientation. Those patterns having the X spacing of less than 110% of the Y spacing is considered as not having a Longitudinal Orientation.

Longitudinal Orientation may be determined by Image Analysis, as discussed in further detail in the measurement section below. The Longitudinal/Transverse Distribution is for extracting the longitudinally continuous and transverse continuous impressions of a pattern by utilizing image analysis, in order to define the distribution of a particular pattern in the longitudinal and transverse direction.

Without being bound by theory, by providing the First Belt Pattern FBP to have a Longitudinal Orientation, the non-elastic region (221) appears coordinated with the Upper Gather Region (220) to enhance the directionality of the longitudinally continuing gathers of the Upper Gather Region (220).

The crotch region (30) herein is provided with a First Crotch Pattern FCP. The First Crotch Pattern FCP may be an Aesthetic Pattern or a Geometric Pattern. The First Crotch Pattern FCP may have a Longitudinal Orientation, according to the measurements herein. Similarly to the First Belt Pattern FBP, the Longitudinal Orientation of the First Crotch Pattern FCP may be measured by Observation and/or determined by Image Analysis. What is described by Longitudinal Orientation, according to measurements herein, is the longitudinally directed impression that the naked eye perceives when observing the repeating visual presentation of a pattern, similar to what was discussed for the First Belt Pattern FBP. Referring to Figure 6A, the crotch region (30) is provided with a First Crotch Pattern FCP of apertures which are visible from the garment facing side, and similar to that of the First Belt Pattern FBP. The First Crotch Pattern FCP also has a Longitudinal Orientation. Referring to Figure 6B, 11D and the measurement method below, the crotch region (30) of Figure 6B is provided with a First Crotch Pattern FCP of apertures which are not being similar to that of the First Belt Pattern FBP. Yet, the apertures of the First Crotch Pattern FCP have a Longitudinal Orientation, thus appear aligned with the First Belt Pattern FBP.

By providing both the First Belt Pattern FBP and the First Crotch Pattern FCP to have Longitudinal Orientation, the non-elastic regions (221) appear coordinated with the crotch region (30) to enhance the integral undergarment like appearance of the article. Such combination of the First Belt Pattern FBP and the First Crotch Pattern FCP is particularly useful for belt-type pants wherein the ring like elastic belt (40) and the central chassis (38) are provided as different parts. By providing both the First Belt Pattern FBP and the First Crotch Pattern FCP to have Longitudinal Orientation, the visibility of the border between the different parts may be alleviated. The First Belt Pattern FBP and First Crotch Pattern FCP may both be a Geometric Pattern.

The First Belt Pattern FBP and the First Crotch Pattern FCP may have a common element. By having a common element, what is meant is that the Basic Unit, according to the measurements herein, of the First Belt Pattern FBP and the First Crotch Pattern FCP are provided the same or similar. For example, the First Belt Pattern FBP and the First Crotch Pattern FCP of Figure 6A have a common element, the common element being the longitudinally linearly lined apertures.

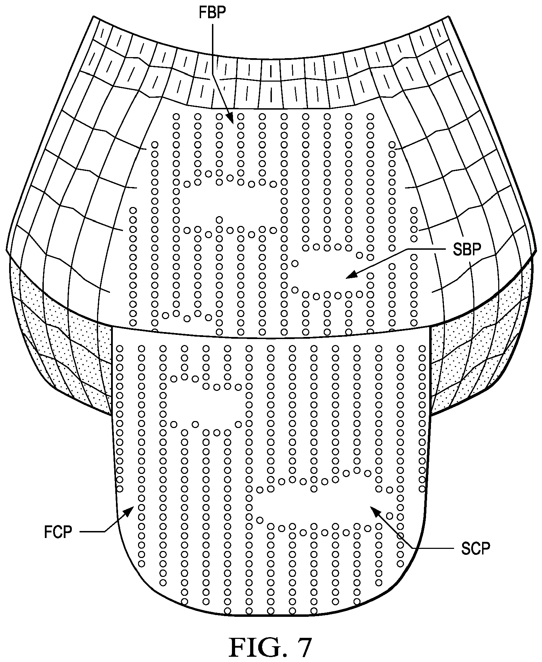

The common element may be expressed in different sizes wherein the Basic Unit is geometrically similar. The common element may be disposed in similar orientation of direction, color, and density. For example, referring to Figure 7, the cloud like patterns of the Second Belt Pattern SBP and Second Crotch Pattern SCP are provided in similar, yet different sizes of the cloud. The cloud like pattern may serve as the common element.

The common element may be provided in different methods. For example, a particular element provided as a Geometric Pattern for the First Belt Pattern FBP may be provided as a printed pattern for the First Crotch Pattern FCP. In another example, a particular element provided as an embossing for the First Belt Pattern FBP may be provided as an aperture for the First Crotch Pattern FCP.

Referring back to Figure 6B, the crotch region (30) may be provided with a First Crotch Pattern FCP of apertures which are different from that of the First Belt Pattern FBP. Yet, when both of the apertures of the First Belt Pattern FBP and the First Crotch Pattern FCP have a Longitudinal Orientation, there is provided an integral appearance. However, in such situation, care may be provided such that the First Crotch Pattern FCP does not interfere with the appearance of the First Belt Pattern FBP, wherein the front and back elastic belts (84, 86) overlap the central chassis (38).

For preventing the interference as discussed above, the front or back elastic belts (84, 86) may be provided to have an Opacity of at least about 25%, or at least about 45%, according to the measurements herein. By providing such Opacity, and providing the First Belt Pattern FBP to have a Longitudinal Orientation, it is difficult to see through the materials configuring the belt materials, such that it is difficult to identify the First Crotch Pattern FCP through the belt materials.

Referring to Figure 2B, for preventing the interference as discussed above, the configuration of the garment facing surface of the central chassis (38) may be adjusted relative to the front and back elastic belts (84, 86). The outer cover layer (42) may be the garment-facing surface in the crotch region (30) and provided with the First Crotch Pattern FCP. The outer cover layer (42) may extend only partly in the longitudinal direction of the front waist panel (52) and the back waist panel (54) to leave the distal parts of the front waist panel (52) and the back waist panel (54) free of the outer cover layer (42). Namely, the longitudinal length of the outer cover layer (42) may be longer than the longitudinal length of the crotch panel (56) and shorter than the longitudinal length of the backsheet (60). By such configuration, the distal parts of the front waist panel (52) and the back waist panel (54) are devoid of the outer cover layer (42). Accordingly, looking at the layers of elements between the garment facing surface and the backsheet (60) of the center chassis (38) of Figure 2B, there exists only a very small overlap region (34) disposed on the waist panel (52) where the outer cover layer (42) is present. The longitudinal length of the overlap region (34) with the front elastic belt (84) and the back elastic belt (86) may be made as short as possible, for example, less than about 20mm, or less than about 15mm, or less than about 10mm, respectively.

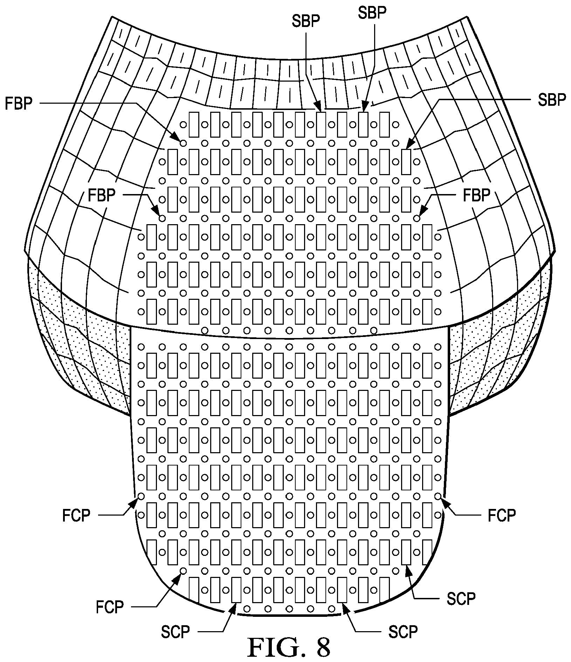

The non-elastic region (221) may further comprise a Second Belt Pattern SBP. The Second Belt Pattern SBP is a different pattern from the First Belt Pattern FBP in either visual presentation or a similar pattern provided in different methods, as discussed above. By similar pattern provided in different methods, what is meant is, for example a First Belt Pattern FBP provided in embossing, and a Second Belt Pattern SBP provided in color matching the embossing. The Second Belt Pattern SBP may be an Aesthetic Pattern or a Geometric Pattern. The Second Belt Pattern SBP may have a common element with the First Belt Pattern FBP or the First Crotch Pattern FCP. The crotch region (30) may further comprise a Second Crotch Pattern SCP. The Second Crotch Pattern SCP is a different pattern from the First Crotch Pattern FCP in either visual presentation or a similar pattern provided in different methods, as discussed above. The Second Crotch Pattern SCP may be an Aesthetic Pattern or a Geometric Pattern. The Second Crotch Pattern SCP may have a common element with the First Belt Pattern FBP or the First Crotch Pattern FCP. The Second Belt Pattern SBP and the Second Crotch Pattern SCP may have a common element. Referring to Figure 7, the First Belt Pattern FBP and the First Crotch Pattern FCP are apertures having Longitudinal Orientation having common element, while the Second Belt Pattern SBP and the Second Crotch Pattern SCP are arrangement of apertures expressing clouds, wherein the clouds do not necessarily have Longitudinal Orientation. Referring to Figure 8, the First Belt Pattern FBP and the First Crotch Pattern FCP are apertures having Longitudinal Orientation having common element, while the Second Belt Pattern SBP and the Second Crotch Pattern SCP are embossings also having Longitudinal Orientation having common element.

The First Belt Pattern FBP, the First Crotch Pattern FCP, the Second Belt Pattern SBP, and the Second Crotch Pattern SCP may each have a Pattern Density, according to measurements herein. The Pattern Density of a particular pattern may be selected in order to provide a particular aesthetic effect. For example, the Pattern Density of the First Belt Pattern FBP and the First Crotch Pattern FCP may be made to have less than about 10 points difference, such that the non-elastic region (221) appears coordinated with the crotch region (30) to enhance the integral undergarment like appearance of the article. For the belt-type pant, the visibility of the border between the different parts may be alleviated. In another example, the Pattern Density may be provided in a gradient which gradually decreases from the distal edge (88) towards the proximal edge (90) and further into the crotch region (30).

Referring to Figure 6A, when the First Belt Pattern FBP and the First Crotch Pattern FCP have common element and are the same or very similar to each other, and further have a Pattern Density difference of less than about 10 points, this may provide a highly coordinated appearance. Such highly coordinated appearance may connote a neat and finished product, while it may also connote less intentionality from the patterns. Referring to Figure 7, by providing a Second Belt Pattern FBP and Second Crotch Pattern SCP to a pattern coordination of Figure 6A, this may help provide accent and/or intentionality to the otherwise highly coordinated appearance of Figure 6A.

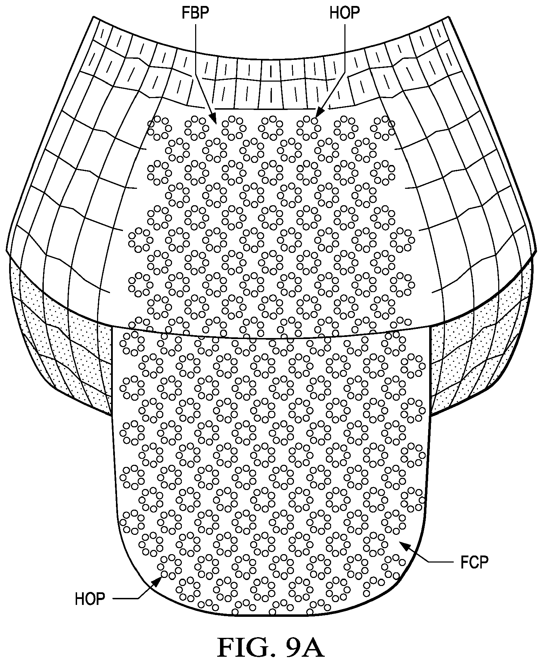

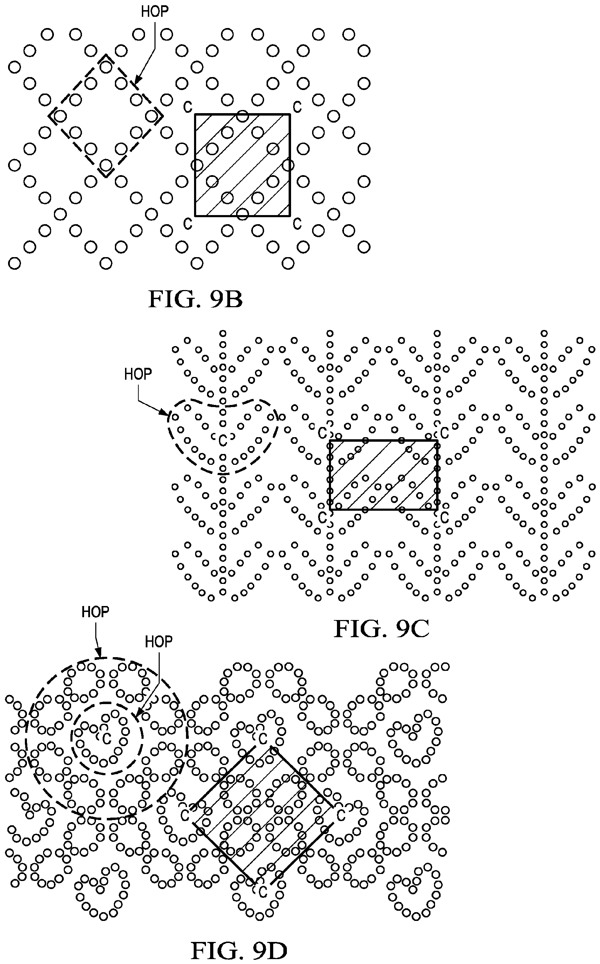

The First Belt Pattern FBP and the First Crotch Pattern FCP may form a High Order Pattern HOP in an area dimension of from about $16mm^2$ to about $200mm^2$ werein the First Belt Pattern FBP and the First Crotch Pattern FCP are Geometric Patterns having a common element, wherein both the First Belt Pattern FBP and the First Crotch Pattern FCP having a Non-directional Orientation, according to the measurements herein. By Non-directional Orientation, what is meant is an impression that the pattern is neither oriented in the longitudinal direction or the transverse direction. The direction orientation of the High Order Pattern HOP is determined by Image Analysis, according to measurements herein. Specifically, a Non-direction Orientation has a Longitudinal Distribution (distribution% at 90 degrees) of less than about 35% as well as a Transverse Distribution (distribution% at 0 degrees) of less than about 35%. Specifically, a Non-direction Orientation may have a Longitudinal Distribution of less than about 15% as well as a Transverse Distribution of less than about 15%. What is meant by High Order Pattern HOP is a pattern which, unlike the randomly repeating unit described above, have inconsistent spacing between the deformations. The inconsistent spacing may be alternating or varying. Because of the inconsistent spacing, the pattern may provide a presentation of a shape that is of a greater dimension than that of the individual deformations. Referring to Figure 9A, the First Belt Pattern FBP as well as the First Crotch Pattern FCP have 7 deformations arranged in a pattern having a circle shape. The deformations may be apertures. Referring to Figure 9B, the First Belt Pattern FBP as well as the First Crotch Pattern FCP have 12 deformations arranged in a diamond shape having an opening in the middle. The deformations may be apertures. Referring to Figure 9C, the First Belt Pattern FBP as well as the First Crotch Pattern FCP have 26 deformations arranged in two leaf like shapes having a stalk connecting them. The deformations may be apertures. Referring to Figure 9D, the First Belt Pattern FBP as well as the First Crotch Pattern FCP have 80 deformations arranged in chain like shapes, while the Second Belt Pattern SBP as well as the Second Crotch Pattern SCP have 16 deformations arranged in a heart like shape surrounded by the chain like First Belt Pattern FBP and First Crotch Pattern FCP, respectively. All such deformations may be apertures. Alternatively, the heart like shape Second Belt Pattern SBP or Second Crotch Pattern SCP may be provided by embossing or printing.

The area dimension of a High Order Pattern HOP is obtained as follows. First, a base pattern of the High Order Pattern HOP is identified. By base pattern, what is meant is, for example, the circle shape of Figure 9A, the diamond shape of Figure 9B, the two leaves plus stalk shape of Figure 9C, the heart shape of Figure 9D, or the chain shape of Figure 9D. Such base pattern may or may not share elements with patterns in the vicinity. The centerpoint of one base pattern is identified as C. Due to the Non-directional Orientation of the High Order Pattern HOP, 4 centerpoints of 4 different High Order Patterns HOP closest to each other may be extracted. The patterns closest to and/or sharing elements with the base pattern are identified. Phantom lines connecting the 4 centerpoints represent a quadrangle, typically a parallelogram, rhomboid or square, and define a HOP Unit. If it is possible to draw more than one type of quadrangle using 4 centerpoints, the quadrangle having the smallest area is identified, and defined as a HOP Unit. The HOP Unit of the present invention has an area of from about $16mm^2$ to about $200mm^2$.

Referring to Figure 9B, the centerpoint of the High Order Pattern HOP may be the opening in the middle and identified as C. The patterns closest to the base pattern are identified. Phantom lines connecting the 4 centerpoints represent a square and define the HOP Unit for the pattern of Figure 9B. Referring to Figure 9C, the centerpoint of the High Order Pattern HOP may be the connecting point of two opposite extending leaf like shapes and identified as C. The patterns closest to the base pattern are identified. Phantom lines connecting the 4 centerpoints represent a square and define the HOP Unit for the pattern of Figure 9C. Referring to Figure 9D, the centerpoint of the High Order Pattern HOP may be the center of the chain like shape and identified as C. The patterns closest to the base pattern are identified. Phantom lines connecting the 4 centerpoints represent a diamond type shape and define the HOP Unit for the pattern of Figure 9D. In Figures 9B, 9C, and 9D, the HOP Unit Area is described in shade, respectively. The individual deformations of Figures 9A through 9D may have a diameter of from about 0.4 mm to about 1.5 mm, and have an area of from about from about $16mm^2$ to about $200mm^2$. By providing the HOP Unit in such area dimension, the pattern is well discerned by the naked eye and the repetition of the patterns are well recognized, providing a high quality aesthetic appearance.

By providing both the First Belt Pattern FBP and the First Crotch Pattern FCP to have a Non-directional Orientation, and further having a common element of a High Order Pattern HOP, the non-elastic region (221) appears coordinated with the crotch region (30) to enhance the integral undergarment like appearance of the article. For the belt-type pant, the visibility of the border between the different parts may be alleviated.

1. Whole Article Force Measurement

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10% and 90% of capacity of the load cell used. The instrument is calibrated according to the manufacturer's instructions. All testing is performed in a room maintained at $23 \pm 2$ °C and $50 \pm 5$ % relative humidity.

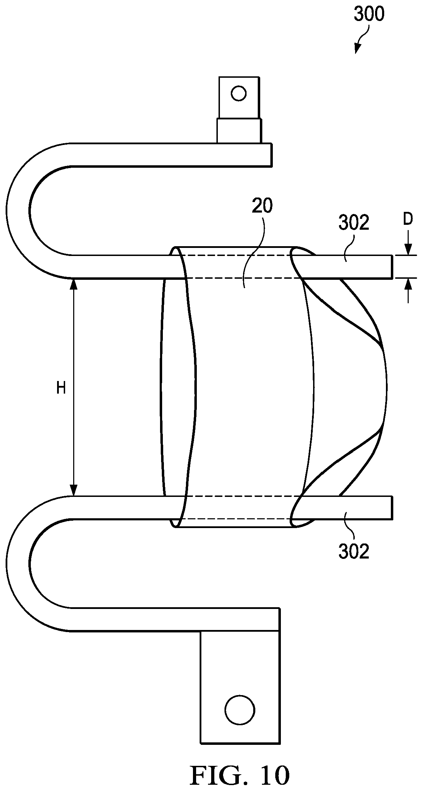

The tensile tester is fitted with hanger-type sample holding fixtures (300) as shown in Figure 10. Each fixture comprises a rigid linear rubber-coated horizontal bar section (302) to prevent sample slippage during testing. The outer bar diameter (including the rubber coating) of the horizontal bar sections is 10.0 mm. The central axes of the horizontal bar sections (302) are configured to remain parallel and in the same vertical plane throughout the test procedure. The gauge circumference is determined by the following equation:

$$\text{Gauge Circumference} = 2 \times (H + D + \pi D/2)$$

where H is the vertical gap between the horizontal bar sections (302), and D is the outer diameter of the bar.

The instrument is set up to go through the following steps:

| | |
|---|---|
| Crosshead Speed | 254.0mm/min |
| Final Load Point | 19.61 N |
| Hold Time | 0 |
| Number of Cycles | 1 |
| Data Acquisition Rate | 50Hz |

An article (20) sample is inserted onto the upper horizontal bar section (302) so that the bar passes through the waist opening and one leg opening of the article. The crosshead is raised until the specimen hangs above the lower bar and does not touch lower bar (302). The load cell is tared and the crosshead is lowered to enable the lower bar (302) to be inserted through the waist opening and other leg opening without stretching the article. The article is adjusted so that the longitudinal centerline LX of the article is in a horizontal plane halfway between the upper and lower bars (302). The center of the side portion in contact with the bar (302) is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the Initial Gauge Circumference at the same speed. The maximum circumference at 19.6N and the force at 70% of the maximum circumference during the loading segment and unloading segment of the test are recorded.

The maximum circumference (mm) at 19.6N is defined as the Full Stretch Circumference W1. The Full Stretch Circumference (mm) $\times$ 0.7 is defined as the 70% Stretch Circumference W2 . The force (N) during the loading segment of the test at 70% Stretch Circumference is defined as the Stretch Circumference Force. The force (N) during the unloading segment of the test at 70% Stretch Circumference is defined as the Fit Circumference Force. Five samples are analyzed and their average are calculated and reported to the nearest 1mm or 0.01N, respectively.

2. Opacity

The opacity of a material, or material combined, is the degree to which light is blocked by that material. A higher opacity value indicates a higher degree of light block by the material. Opacity may be measured using a 0° illumination / 45° detection, circumferential optical geometry, spectrophotometer with a computer interface such as the HunterLab LabScan XE running Universal Software (available from Hunter Associates Laboratory Inc., Reston, VA). Instrument calibration and measurements are made using the standard white and black calibration plates provided by the vendor. All testing is performed in a room maintained at about 23 ± 2 °C and about 50 ± 5 % relative humidity.

The spectrophotometer is configured for the XYZ color scale, D65 illuminant, 10° standard observer, with UV filter set to nominal. The instrument is standardized according to the manufacturer's procedures using the 44.45 mm (1.750 inch) area view. After calibration, the software is set to the Y opacity procedure which prompts the operator to cover the sample with either the white or black calibration tile during the measurement.

To obtain a sample, the front or back elastic belt is removed from the remainder of the article by removing the center chassis by hand after applying cold spray, such as La Pointique Int'l Ltd. Cold spray 829, to the adhesive connecting the belt and center chassis. The belt thus removed from the center chassis is opened at the side seams by scissors. The non-elastic region of the belt is cut into a 101.6 mm by 101.6 mm portion using scissor for analysis. Samples are pre-conditioned at 23 °C ± 2 C° and 50% ± 5% relative humidity for two hours prior to testing.

Place sample over the measurement port. The sample should completely cover the port with the surface corresponding to the garment-facing surface of the article directed toward the port. Cover the specimen with the white standard plate. Take a reading, then remove the white tile and replace it with the black standard tile without moving the specimen. Obtain a second reading, and calculate the opacity as follows:

$$\text{Opacity} = (\text{Y value}_{(\text{black backing})} \quad / \quad \text{Y value}_{(\text{white backing})}) \quad \text{x } 100$$

A total of three belts of similar region are analyzed and their opacity results recorded. Calculate and report the average opacity to the nearest 0.1%.

3. Longitudinal Orientation by Observation

This measurement is provided for a visible randomly repeating pattern, which is defined as a visually recognizable pattern by the naked eye when observed from a distance of 10cm. Referring to Figures 11A - 11D, identify a Basic Unit which is a minimum complete unit of the pattern that repeats to create the pattern and capable of surrounding a specific area. Within the Basic Unit, the closest adjacent deformation or printing in the longitudinal direction and the transverse direction are identified. The spacing between the closest adjacent deformation/printing in the longitudinal direction (Y) and the same in the transverse direction (X) are measured by a scale (unit: 0.1 mm). The Y spacing and/or X spacing may be negative, as is the case for the Y spacing in Figure 11C.

Those patterns having the X spacing greater than the Y spacing by at least 10% are considered having a Longitudinal Orientation. Five portions of the same pattern are analyzed and their average are calculated and reported to the nearest %.

4. HOP Unit Area

This measurement is provided for a High Order Pattern HOP. A HOP is a Geometric Pattern visually recognizable by the naked eye when observed from a distance of 10cm, and having inconsistent spacing between the deformations. The inconsistent spacing may be alternating or varying. The repetition of a HOP made of a number of deformations is identified by observation. Referring to Figures 9A-9D, the HOP of Figure 9A is a circle, Figure 9B is a square, Figure 9C is a leaf with stalk, and Figure 9D is a heart surrounded by chains. The center of one HOP is identified as C. Such HOP is the base pattern. Other HOPs closest to, or sharing elements with, the base pattern are identified and extracted. From those HOPs in the vicinity, 4 centerpoints of closest distance are identified. Phantom lines connecting the 4 centerpoints represent a quadrangle. If it is possible to draw more than one type of quadrangle using 4 centerpoints, the quadrangle having the smallest area is identified, and defined as a HOP Unit. The sides and/or diagonals of the quadrangle are measured by a JIS certified metal scale or equivalent, and the area of the quadrangle is calculated.

Five HOP Units of the same pattern are analyzed, and reported to the closest 1mm$^2$.

5. Image Analysis

5-1. Sample preparation

An article (20) sample is mounted on a rigid plastic plate which has an appropriate size which enables mounting the elastic belt (40) of the sample in a state stretched by 65% to 90% of its Full Stretch Circumference W1. For measurement of the article samples of Examples 1-2, a rigid plastic plate having a dimension of 250mm in the transverse direction and a thickness of 4mm was used. Further, a stretching block is inserted in the sample between the front side and the plastic plate. Regardless of the size of the sample, the stretching block has a dimension of 110mm in the transverse direction, 170mm in the longitudinal direction, and thickness of 25mm. The stretching block is inserted to the center of the article such that any elastics on the central chassis (38) are in a substantially stretched state.

## 5-2. Image acquisition

The sample prepared above is placed on a non-reflective black background plate horizontally with the front side facing up. A Canon camera (CanonEO2 6D Mark 2) with lens (EF 24-105mm f/4L IS2 USM) or equivalent is placed directly vertically above the sample in a length of 1050mm. Two bar lights (Smart Vision Lights LHF 300 or equivalent) are placed 650mm away from the sample in the transverse direction, 300mm away from the sample in the vertical direction, wherein the surface of the light is faced in an angle of $45 \pm 6$ degrees from the horizontal direction, and the longer dimension of the bar light is placed in parallel with the longitudinal axis of the sample. The focal length of the camera is set to 64mm. The image acquisition settings are; ISO: 400, F: 5.0, exposure time: 1/160seconds

## 5-3. Image analysis for Pattern Density

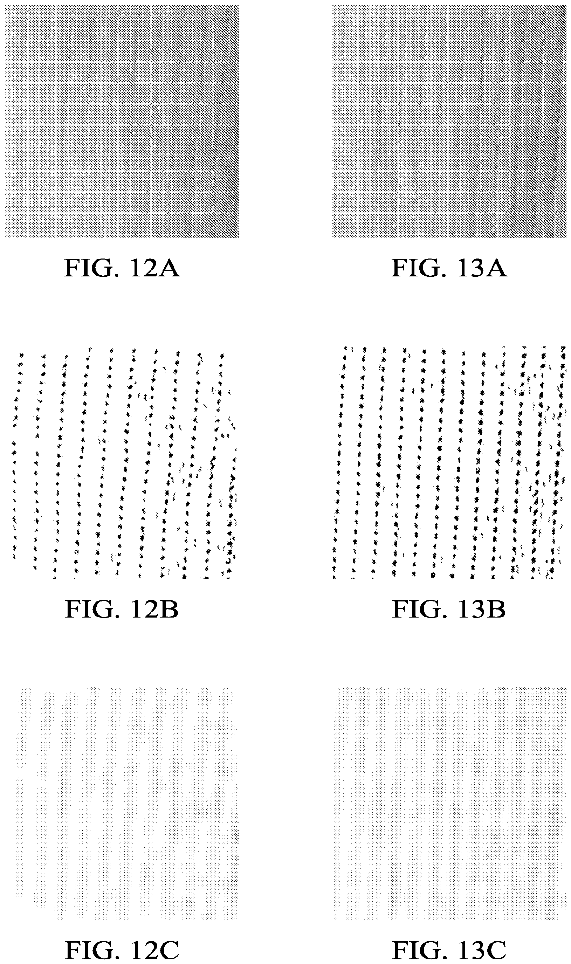

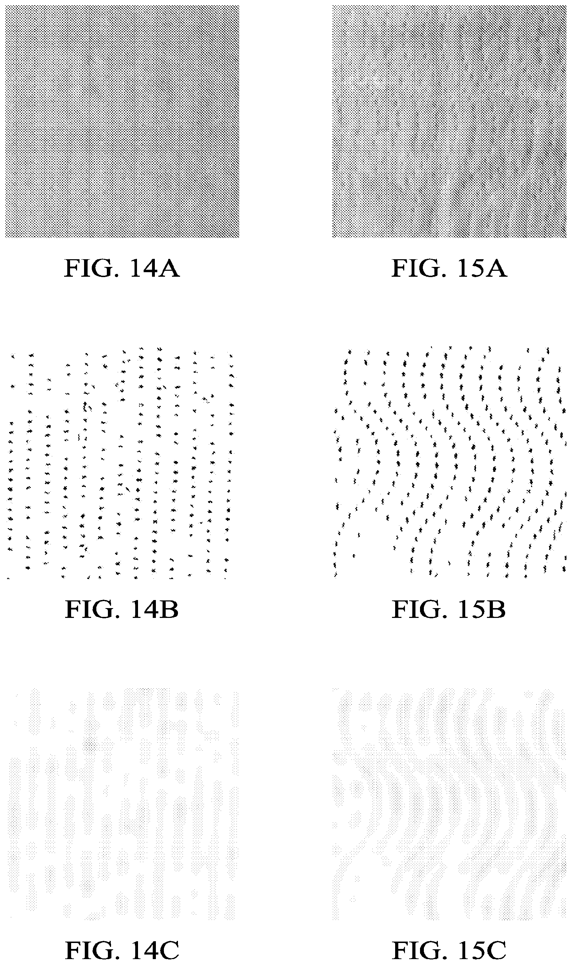

a) The above acquired images are imported into ImageJ software (version. 1.52h, National Institute of Health, USA) or equivalent, and converted into 8 bit.
b) Set the scale referencing to the rigid plastic plate.
c) The analyzing region of the First Belt Pattern (FBP) and First Crotch Pattern (FCP) are cropped out simultaneously as a 50mm × 50mm square each, and side by side, from nearly the transverse center of the article, while avoiding regions which have wrinkles, shadows, or high reflection. The images obtained by this step were as such: Figure 12A for FBP of Example 1, Figure 13A for FCP of Example 1, Figure 14A for FBP of Example 2, and Figure 15A for FCP of Example 2. This order of Figures (12, 13, 14, 15) for the respective patterns will be the same for the remaining images described below.
d) The cropped images are filtered using the "FFT bandpass filter" in ImageJ with filter large structure down to 10 pixels, filter small structure up to 3 pixels with check marks on the "function of auto scale after filtering" and "surtulation when autoscaling".
e) The filtered images obtained in step d) are converted to black & white color using threshold at 70 gray value.
f) An ImageJ built-in plugin called "particle analysis" is applied to the threshold images obtained in step e) for filtering out particles having a size of less than 0.2mm$^2$, set "Show" pull down menu to "Masks" to create cleaned pattern images and check mark on the summary table. The cleaned pattern images obtained by this step for Examples 1 and 2 were Figures 12B - 15B. The pattern density is defined as % Area from the summary table. Five samples are analyzed and their average are calculated and reported to the nearest 0.1 points. 5-4. Image analysis for Longitudinal Distribution / Transverse Distribution
g) The cleaned pattern images obtained in step f) are T filtered using the "Gaussian Blur" filter in ImageJ with a Sigma (radius) of 1mm. The images obtained by this step for Examples 1 and 2 were Figures 12C - 15C.
h) To calculate the orientation distribution, an ImageJ built-in plugin called "directionality" is applied to the blur filtered images obtained in step g) to calculate the orientation distribution. The analysis parameters used are: Method: Fourier components; Nbins: 5, histogram start: -45; histogram end: 135 with check mark at the Display table.
i) Obtain directional distribution from the output table. The output table contains distribution of each angle -45°, 0°, 45°, 90° and 135°. Transversal Distribution is defined as the distribution% at 0°. Longitudinal Distribution is defined as the distribution% at 90°. Five samples are analyzed and their average are calculated and reported to the nearest 0.01 points.

EXAMPLES

Examples 1 and 2 were created by having the belt configuration of Figure 2A, the planning of First Belt Pattern FBP and First Crotch Pattern FCP as in Figures 6A and 6B, and elastic/bonding profile as in Table 1, respectively. Actual photograph images of Example 1 FBP, Example 1 FCP, Example 2 FBP, and Example 2 FCP, are provided as Figures 12A, 13A, 14A, and 15A, respectively. The Longitudinal/Transverse Distribution and Pattern Density of Examples 1 and 2 were obtained, as in Table 2.

Table 1

| | Dtex / elongation% / number of elastic members |
|---|---|
| Front waist zone | 470Dtex / 160% / 4 with elastic pitch of 6mm |
| Front distal tummy zone | 470Dtex / 160% / 2 |
| | 470Dtex / 230% / 2 with tummy cut (*1) |
| Front proximal tummy zone | 940Dtex / 230% / 8 with tummy cut (*1) |
| Front leg zone | 470Dtex / 130% / 2 with tummy cut (*1) |
| Back waist zone | 470Dtex / 160% / 4 with elastic pitch of 6mm |
| Back distal tummy zone | 940Dtex / 160% / 4 with elastic pitch of 6mm |
| Back proximal tummy zone | 470Dtex / 230% / 4 |
| | 470Dtex / 270% / 4 with elastic pitch of 12mm and tummy cut (*1) |
| Back leg zone | 470Dtex / 270% / 2 with tummy cut (*1) |
| Outer sheet material | Air-through carded nonwoven made by Beijing Dayuan having basis weight of 20gsm |
| Outer sheet aperture minus radius | 0.45 - 0.55mm |
| Outer sheet DF1 | 3.8mm |
| Outer sheet DF2 | 2mm |
| VG1 | 7mm |
| VG2 | 1mm |
| (*1) "Tummy cut" in Table 1 refers to removal of elasticity at the transverse central area of elastic strands resulting in 68% effective length of elasticity. | |

Table 2

| | Example 1 | Example 2 |
|---|---|---|
| FBP Longitudinal Distribution (%) | 90 | 55 |
| FBP Transverse Distribution (%) | 2 | 5 |
| FBP Pattern Density (%) | 7.8 | 4.4 |
| FCP Longitudinal Distribution (%) | 86 | 51 |
| FCP Transverse Distribution (%) | 2 | 11 |
| FCP Pattern Density (%) | 5.3 | 5.2 |

The article of Examples 1 and 2 provide aesthetically pleasing integral appearance. The article of Examples 1 and 2 also have improved stretchability for ease of application, improved fit for preventing sagging, improved comfort and softness, and improved breathability for skin health.

Show Test based on Synthesized Visual

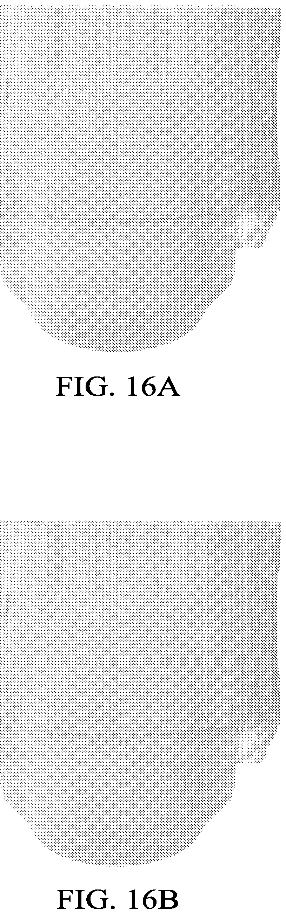

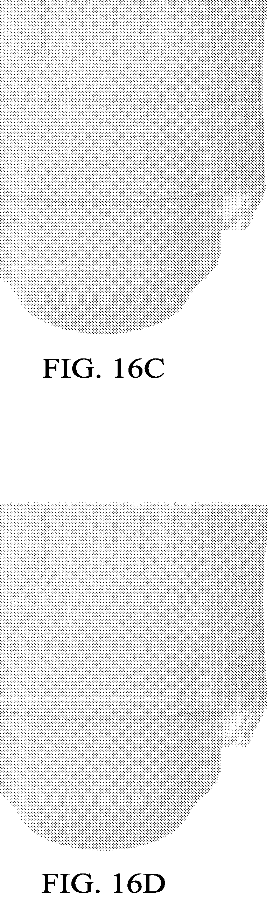

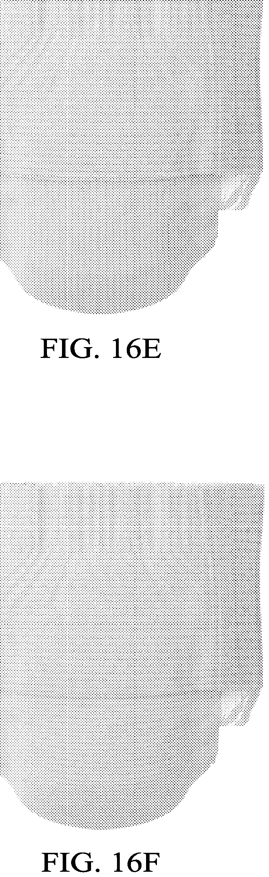

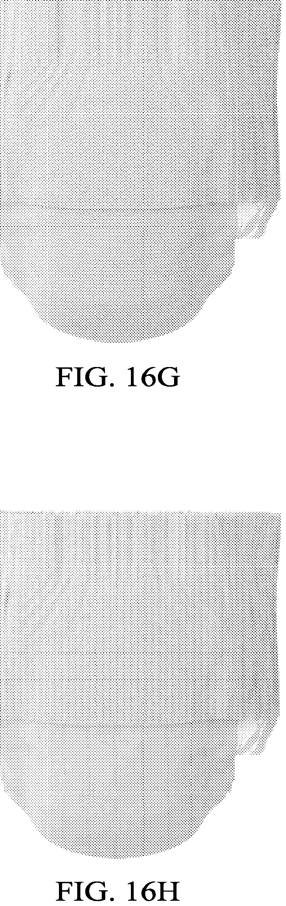

Examples A - H were created as synthetic photographs of pant type articles having an appearance seen from the front of the article as in Figures 16A - 16H, respectively. All deformations are apertures having an approximate circle shape having an aspect ratio of within 2. The synthetic photographs were created to present how the article appears in the worn state, and having the following characteristics. Examples C and D have patterns which are High Order Patterns having a HOP Unit Area of 79mm$^2$ and 20mm$^2$, respectively.

Table 3

| Example | A | B | C | D | E | F | G | H |
|---|---|---|---|---|---|---|---|---|
| FBP Longitudinal Distribution (%) | 97 | 97 | 13 | 5 | 1 | 1 | 56 | 97 |
| FBP Transverse Distribution (%) | 1 | 1 | 13 | 5 | 97 | 97 | 13 | 1 |
| FCP Longitudinal Distribution (%) | 97 | 53 | 13 | 5 | 97 | 1 | 13 | 95 |
| FCP Transverse Distribution (%) | 1 | 1 | 13 | 5 | 1 | 97 | 56 | 1 |
| FBP Pattern Density (%) | 20.3 | 20.3 | 16.6 | 8.8 | 20.3 | 20.3 | 22.1 | 26 |
| FCP Pattern Density (%) | 20.3 | 18.6 | 16.6 | 8.8 | 20.3 | 20.3 | 22.1 | 5.7 |

panelists who were caregivers of babies using pant diapers, mostly of Size 4 or 5 (L or XL size), and having a mixture of usage experience of major brands were recruited. The panelists were shown all of the visuals for Examples A-H and asked to rate the values as found in Table 4 below against those values using the 5 ratings, which were scored as such: "Poor"=0, "Fair"=25, "Good"=50, "Very Good"=75, and "Excellent"=100. The scores were averaged.

Table 4

| Example | A | B | C | D | E | F | G | H |
|---|---|---|---|---|---|---|---|---|
| Overall | 36 | 45 GH(*1) | 70 ABEFG | 58 ABEFGH | 34 | 38 | 29 | 29 |
| Intentionally selected pattern | 26 | 48 AEGH | 76 ABDEFGH | 56 AEFGH | 26 | 33 | 23 | 30 |

(*1) The markings of Example numbers after the scores indicate "statistically significantly better" against the marked Example at 90% confidence level. For example, "Overall" rating of Example B was statistically significantly better than each of Examples G and H.

According to the test above, Examples B, C, and D which meet the requirements of the present invention have statistically significant overall preference over at least some of Examples E through H which do not meet the requirements of the present invention.

**Claims**

1. A wearable article (20) in the form of pants, the wearable article being continuous in a longitudinal direction and a transverse direction comprising a front elastic belt (84), a back elastic belt (86), a crotch region (30), a waist opening, and a pair of leg openings; the crotch region (30) extending longitudinally between the front elastic belt (84) and the back elastic belt (86);

   each of the front elastic belt (84) and the back elastic belt (86) being a laminate comprising an inner sheet (94), an outer sheet (92), and an elastic member (96) running in the transverse direction, and the front elastic belt (84) and the back elastic belt (86) being discontinuous with each other in the crotch region (30);
   the front elastic belt (84) and back elastic belt (86) having a transverse dimension of LW, the smaller longitudinal dimension of the front elastic belt (84) or the back elastic belt (86) having a dimension of LS, wherein from about 10% to about 40% of LW, and from about 15% to about 75% of LS, preferably from about 30% to about 70% of LS, is removed of its elastic activity, wherein the region of the front elastic belt (84) and the back elastic belt (86) removed of its elastic activity defines a non-elastic region (221); the front and back non-elastic regions have a First Belt Pattern FBP and the crotch region (30) has a First Crotch Pattern FCP;
   wherein the First Belt Pattern FBP and the First Crotch Pattern FCP are both a Geometric Pattern, and the First Belt Pattern FBP and the First Crotch Pattern FCP both have a Longitudinal Orientation, according to the measurements herein, and wherein

   a) either the First Belt Pattern FBP and the First Crotch Pattern FCP are formed by apertures having a minor radius of at least about 0.1mm; or
   b) wherein the First Belt Pattern FBP and the First Crotch Pattern FCP are embossings.

**2.** The article (20) of Claim 1 wherein the First Belt Pattern FBP and the First Crotch Pattern FCP have a common element.

**3.** The article (20) of Claim 1 wherein the First Belt Pattern FBP and the First Crotch Pattern FCP are different.

**4.** The article (20) of any of the preceding claims wherein the First Belt Pattern FBP is aligned in a plurality of lines extending in the longitudinal direction, wherein the lines are spaced apart from each other with a transverse pitch DF1 of from about 2 mm to about 15 mm.

**5.** The article (20) of any of the preceding claims wherein the First Crotch Pattern FCP is aligned in a plurality of wavy lines extending in the longitudinal direction.

**6.** The article (20) of any of the preceding claims wherein the non-elastic region (221) further comprises a Second Belt Pattern SBP which is a Geometric Pattern.

**7.** The article (20) of any of the preceding claims wherein the crotch region (30) further comprises a Second Crotch Pattern SCP which is a Geometric Pattern.

**8.** The article (20) of any of the preceding claims wherein the non-elastic region (221) further comprises a Second Belt Pattern SBP which is an Aesthetic Pattern, the crotch region (30) further comprises a Second Crotch Pattern SCP which is an Aesthetic Pattern, wherein the Second Belt Pattern SBP and the Second Crotch Pattern SCP have a common element.

**9.** The article (20) of any of the preceding claims wherein the First Belt Pattern FBP has a Pattern Density and the First Crotch Pattern FCP has a Pattern Density, wherein the difference between the Pattern Density of the First Belt Pattern FBP and that of the First Crotch Pattern FCP is less than about 10 points according to the measurements herein.

**10.** The article (20) of Claim 1, option a), wherein at least one of the front elastic belt (84) and the back elastic belt (86) have an opacity of at least about 25%.

**11.** The article (20) of any of the preceding claims wherein the entire garment facing surface of the front elastic belt (84) and the back elastic belt (86) have the First Belt Pattern FBP.

**12.** The article (20) of any of the preceding claims wherein the First Crotch Pattern FCP is provided on an outer cover layer (42), wherein the outer cover layer (42) extends and overlaps with the front elastic belt (84) and the back elastic belt (86) for less than about 20mm, respectively, in the longitudinal direction.


**Patentansprüche**

**1.** Tragbarer Artikel (20) in der Form von Höschen, wobei der tragbare Artikel in einer Längsrichtung und einer Querrichtung ununterbrochen ist, umfassend einen vorderseitigen Gummibandbund (84), einen rückseitigen Gummibandbund (86), einen Schrittbereich (30), eine Taillenöffnung und ein Paar Beinöffnungen; wobei sich der Schrittbereich (30) zwischen dem vorderseitigen Gummibandbund (84) und dem rückseitigen Gummibandbund (86) der Länge nach erstreckt;

wobei jedes des vorderseitigen Gummibandbundes (84) und des rückseitigen Gummibandbundes (86) ein Laminat ist, umfassend eine Innenlage (94), eine Außenlage (92) und ein Gummibandglied (96), das in der Querrichtung verläuft, und der vorderseitige Gummibandbund (84) und der rückseitige Gummibandbund (86) in dem Schrittbereich (30) miteinander unterbrochen sind; wobei der vorderseitige Gummibandbund (84) und der rückseitige Gummibandbund (86) eine Querabmessung von LW aufweisen, die kleinere Längsabmessung des vorderseitigen Gummibandbundes (84) oder des rückseitigen Gummibandbundes (86) eine Abmessung von LS aufweist, wobei von etwa 10 % bis etwa 40 % von LW und von etwa 15 % bis etwa 75 % von LS, vorzugsweise von etwa 30 % bis etwa 70 % von LS, von seiner Gummibandaktivität befreit wird, wobei der Bereich des vorderseitigen Gummibandbundes (84) und des rückseitigen Gummibandbundes (86), der von seiner Gummibandaktivität befreit ist, einen nichtelastischen Bereich (221) definiert; der vorderseitige und der rückseitige nichtelastische Bereich ein Erstes Bundmuster FBP aufweisen und der Schrittbereich (30) ein Erstes Schrittmuster FCP aufweist;
wobei das Erste Bundmuster FBP und das Erste Schrittmuster FCP beide geometrisches Muster sind, und das Erste Bundmuster FBP und das Erste Schrittmuster FCP beide eine Längsausrichtung, nach den hierin beschriebenen Messungen, aufweisen, und wobei

a) entweder das Erste Bundmuster FBP und das Erste Schrittmuster FCP durch Öffnungen, die einen kleineren Radius von mindestens etwa 0,1 mm aufweisen, gebildet werden; oder
b) wobei das Erste Bundmuster FBP und das Erste Schrittmuster FCP Prägungen sind.

2. Artikel (20) nach Anspruch 1, wobei das erste Erste Bundmuster FBP und das Erste Schrittmuster FCP ein gemeinsames Element aufweisen.

3. Artikel (20) nach Anspruch 1, wobei das erste Erste Bundmuster FBP und das Erste Schrittmuster FCP unterschiedlich sind.

4. Artikel (20) nach einem der vorstehenden Ansprüche, wobei das Erste Bundmuster FBP in einer Vielzahl von Linien ausgerichtet ist, die sich in der Längsrichtung erstrecken, wobei die Linien mit einem querverlaufenden Grad DF1 von etwa 2 mm bis etwa 15 mm voneinander beabstandet sind.

5. Artikel (20) nach einem der vorstehenden Ansprüche, wobei das Erste Schrittmuster FCP in einer Vielzahl von Wellenlinien ausgerichtet ist, die sich in der Längsrichtung erstrecken.

6. Artikel (20) nach einem der vorstehenden Ansprüche, wobei der nichtelastische Bereich (221) ferner ein Zweites Bundmuster SBP umfasst, das ein geometrisches Muster ist.

7. Artikel (20) nach einem der vorstehenden Ansprüche, wobei der Schrittbereich (30) ferner ein Zweites Schrittmuster SCP umfasst, das ein geometrisches Muster ist.

8. Artikel (20) nach einem der vorstehenden Ansprüche, wobei der nichtelastische Bereich (221) ferner ein Zweites Bundmuster SBP umfasst, das ein Ästhetikmuster ist, wobei der Schrittbereich (30) ferner ein Zweites Schrittmuster SCP umfasst, das ein Ästhetikmuster ist, wobei das Zweite Bundmuster SBP und das Zweite Schrittmuster SCP ein gemeinsames Element aufweisen.

9. Artikel (20) nach einem der vorstehenden Ansprüche, wobei das Erste Bundmuster FBP eine Musterdichte aufweist und das Erste Schrittmuster FCP eine Musterdichte aufweist, wobei die Differenz zwischen der Musterdichte des Ersten Bundmusters FBP und der des Ersten Schrittmusters FCP weniger als etwa 10 Punkte nach den Messungen hierin beträgt.

10. Artikel (20) nach Anspruch 1, Option a), wobei mindestens eines von dem vorderseitigen Gummibandbund (84) und dem rückseitigen Gummibandbund (86) eine Trübung von mindestens etwa 25 % aufweist.

11. Artikel (20) nach einem der vorstehenden Ansprüche, wobei die gesamte bekleidungsseitige Oberfläche des vorderseitigen Gummibandbundes (84) und des rückseitigen Gummibandbundes (86) das Erste Bundmuster FBP aufweisen.

12. Artikel (20) nach einem der vorstehenden Ansprüche, wobei das Erste Schrittmuster FCP an einer Außenmantelschicht (42) bereitgestellt ist, wobei sich die Außenmantelschicht (42) in der Längsrichtung mit dem vorderseitigen Gummibandbund (84) und dem rückseitigen Gummibandbund (86) für weniger als etwa 20 mm erstreckt und überlappt.

**Revendications**

1. Article portable (20) sous la forme de pantalon, l'article portable étant continu dans une direction longitudinale et une direction transversale comprenant une ceinture élastique avant (84), une ceinture élastique arrière (86), une région d'entrejambe (30), une ouverture de taille, et une paire d'ouvertures de jambe ; la région d'entrejambe (30) s'étendant longitudinalement entre la ceinture élastique avant (84) et la ceinture élastique arrière (86) ;

chacune parmi la ceinture élastique avant (84) et la ceinture élastique arrière (86) étant un stratifié comprenant une feuille interne (94), une feuille externe (92), et un élément élastique (96) allant dans la direction transversale, et la ceinture élastique avant (84) et la ceinture élastique arrière (86) étant discontinues l'une avec l'autre dans la région d'entrejambe (30) ;

la ceinture élastique avant (84) et la ceinture élastique arrière (86) ayant une dimension transversale de LW, la plus petite dimension longitudinale de la ceinture élastique avant (84) ou de la ceinture élastique arrière (86) ayant une dimension de LS, dans lequel d'environ 10 % à environ 40 % de LW, et d'environ 15 % à environ 75 % de LS, de préférence d'environ 30 % à environ 70 % de LS, est retirée de son activité élastique, dans lequel la région de la ceinture élastique avant (84) et de la ceinture élastique arrière (86) retirée de son activité élastique définit une région non élastique (221) ; les régions non élastiques avant et arrière ont un premier motif de ceinture FBP et la région d'entrejambe (30) a un premier motif d'entrejambe FCP ;

dans lequel le premier motif de ceinture FBP et le premier motif d'entrejambe FCP sont l'un et l'autre un motif géométrique, et le premier motif de ceinture FBP et le premier motif d'entrejambe FCP ont l'un et l'autre une orientation longitudinale, selon les mesures ici, et dans lequel

a) ou bien le premier motif de ceinture FBP et le premier motif d'entrejambe FCP sont formés par des ouvertures ayant un rayon mineur d'au moins environ 0,1 mm ; ou

b) dans lequel le premier motif de ceinture FBP et le premier motif d'entrejambe FCP sont des gaufrages.

2. Article (20) selon la revendication 1 dans lequel le premier motif de ceinture FBP et le premier motif d'entrejambe FCP ont un élément commun.

3. Article (20) selon la revendication 1 dans lequel le premier motif de ceinture FBP et le premier motif d'entrejambe FCP sont différents.

4. Article (20) selon l'une quelconque des revendications précédentes dans lequel le premier motif de ceinture FBP est aligné dans une pluralité de lignes s'étendant dans la direction longitudinale, dans lequel les lignes sont espacées l'une de l'autre avec un pas transversal DF1 d'environ 2 mm à environ 15 mm.

5. Article (20) selon l'une quelconque des revendications précédentes dans lequel le premier motif d'entrejambe FCP est aligné dans une pluralité de lignes ondulées s'étendant dans la direction longitudinale.

6. Article (20) selon l'une quelconque des revendications précédentes dans lequel la région non élastique (221) comprend en outre un second motif de ceinture SBP qui est un motif géométrique.

7. Article (20) selon l'une quelconque des revendications précédentes dans lequel la région d'entrejambe (30) comprend en outre un second motif d'entrejambe SCP qui est un motif géométrique.

8. Article (20) selon l'une quelconque des revendications précédentes dans lequel la région non élastique (221) comprend en outre un second motif de ceinture SBP qui est un motif esthétique, la région d'entrejambe (30) comprend en outre un second motif d'entrejambe SCP qui est un motif esthétique, dans lequel le second motif de ceinture SBP et le second motif d'entrejambe SCP ont un élément commun.

9. Article (20) selon l'une quelconque des revendications précédentes dans lequel le premier motif de ceinture FBP a une densité de motif et le premier motif d'entrejambe FCP a une densité de motif, dans lequel la différence entre la densité de motif du premier motif de ceinture FBP et celle du premier motif d'entrejambe FCP est inférieure à environ 10 points selon les mesures ici.

10. Article (20) selon la revendication 1, option a), dans lequel au moins l'une parmi la ceinture élastique avant (84) et la ceinture élastique arrière (86) ont une opacité d'au moins environ 25 %.

11. Article (20) selon l'une quelconque des revendications précédentes dans lequel toute la surface tournée vers le vêtement de la ceinture élastique avant (84) et de la ceinture élastique arrière (86) ont le premier motif de ceinture FBP.

12. Article (20) selon l'une quelconque des revendications précédentes dans lequel le premier motif d'entrejambe FCP est fourni sur une couche de protection externe (42), dans lequel la couche de protection externe (42) s'étend et chevauche la ceinture élastique avant (84) et la ceinture élastique arrière (86) pour moins d'environ 20 mm, respectivement, dans la direction longitudinale.

FIG. 1A

FIG. 1B

FIG. 2A

EP 3 923 888 B1

FIG. 2B

**FIG. 3A**

234

96

234

## FIG. 3B

234          234          92          234

VG2          96          VG1

## FIG. 4A

234          234          92          234

VG2          96

## FIG. 4B

DF2

DF1

**FIG. 5A**

**FIG. 5B**          **FIG. 5C**

FBP

FCP

**FIG. 6A**

FBP

FCP

FIG. 6B

FIG. 7

FIG. 8

FIG. 9A

**FIG. 9B**

**FIG. 9C**

**FIG. 9D**

FIG. 10

FIG. 11A

FIG. 11B

**FIG. 11C**

**FIG. 11D**

FIG. 12A

FIG. 13A

FIG. 12B

FIG. 13B

FIG. 12C

FIG. 13C

FIG. 14A

FIG. 15A

FIG. 14B

FIG. 15B

FIG. 14C

FIG. 15C

FIG. 16A

FIG. 16B

FIG. 16C

FIG. 16D

FIG. 16E

FIG. 16F

FIG. 16G

FIG. 16H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200617718 A
- WO 2016101197 A
- US 201723183 B
- WO 2016029373 A
- US 2018289563 A
- WO 2017212857 A

- JP 3217273 B
- WO 2016048337 A
- JP 2015128573 B
- WO 2017212856 A
- WO 2011087503 A